# EUROPEAN PATENT SPECIFICATION

(11) **EP 4 313 221 B1**
(45) Date of publication and mention of the grant of the patent: **14.10.2026**
(21) Application number: 22719803.3
(22) Date of filing: 23.03.2022
(51) Int. Cl.: A61M 11/00, A61M 11/02, A61M 15/00

(54) **AN INHALER MONITORING DEVICE**
INHALATORÜBERWACHUNGSVORRICHTUNG
DISPOSITIF DE SURVEILLANCE D'INHALATEUR

(30) Priority: 24.03.2021 GB 202104153
(43) Date of publication of application: 07.02.2024
(73) Proprietor: Respiratory Analytics, Derry BT47 6WD (GB)
(72) Inventor: MCDAID, Liam, Derry BT476WD (GB); KELLY, Susan, Derry BT476WD (GB); HARKIN, Jim, Derry BT47 6WD (GB)
(74) Representative: Lewis Silkin LLP
(86) International application number: PCT/EP2022/057637
(87) International publication number: WO 2022/200434

(56) References cited:
- WO-A1-2011/083377
- WO-A1-2016/009202
- WO-A1-2020/219012

## Description

### Field of the Invention

This invention relates to an inhaler monitoring device, an inhaler apparatus and an inhaler monitoring system which are operable to provide optimum usage of inhalers, in particular metered dose inhalers.

### Background to the Invention

A widely used method for delivery of medication to treat asthma, Chronic Obstructive Pulmonary Disease (COPD) and other respiratory diseases is by MDI (Metered Dose Inhaler). This method of delivery requires a co-ordinated series of actions by the patient to ensure that the medication within the aerosol from the MDI is deposited correctly, deep in the airways (deposition).

The most commonly used method of improving the efficacy of medication delivery from MDI is the use of a Valved-Holding-Chamber (VHC) or Spacer device in conjunction with the MDI. The process of using a conventional MDI/spacer device combination requires instruction from a Health Care Professional (HCP) to users on performing a series of actions that are challenging both technically and, in their co-ordination, and sequencing. Evidence shows that poor user technique is a global problem which is thought to be caused by difficulty in mastering the correct inhalation technique, remembering the series of steps, and poor user engagement and insight into the benefits of optimal inhalation technique.

MDI device combinations with a data capture device comprising integrated sensors that can capture data indicative of medication adherence have recently become available, e.g. smart inhaler, Propellor Health, CapMedic, PuffClicker. These inventions attach directly to the MDI and send user information to apps. However, they do not attach to a spacer so they cannot enable the full monitoring and subsequent display to users of all essential inhalation steps to allow optimal medication deposition to control respiratory symptoms.

Data analysis of sensor information to provide informed user and HCP feedback, and the dynamic setting of adherence performance metrics, is needed to ensure a data driven, personalised approach towards optimal medication utilisation with the purpose of controlling respiratory symptoms.

WO2011/083377 describes a respiratory drug delivery device having a feedback compliance device.

WO2020/219012 discloses a nebulizer monitoring device and methods of monitoring a user's nebulizer use using the monitoring device. The monitoring device provides measurements of flow, humidity, temperature and movement by sensors disposed in or with the nebulizer to monitor a user's real time use of the nebulizer. The device aids in facilitating compliance of nebulizer use according to a prescribed therapy regime as well as providing real time monitoring of a user and onsets of exacerbations.

The present disclosure relates to one or more intelligent algorithms embedded within a data capture device and/ or central server that monitors sensor information to provide this personalised data. Accordingly, it is a desire of the present invention to overcome the deficiencies of the prior art mentioned above.

### Summary of the Invention

The invention is defined in the appended independent claim 1 defining an inhaler monitoring system and independent claim 13 defining a method for monitoring inhaler technique competence. Preferred embodiments are matter of the dependent claims.

A first aspect of the present disclosure provides an inhaler monitoring device, the inhaler monitoring device comprising:
A body comprising an inlet and an outlet which are in fluid communication with respect to one another to define a channel therebetween;
Wherein the inlet is for coupling to an inhaler for dispensing a medication and the outlet is for coupling to a spacer through which a user can inhale the dispensed medication from the inhaler;
one or more sensors which are configured to measure one or more inhalation characteristics when the user inhales the dispensed medication from the inhaler through the inhaler monitoring device via the spacer in-use;
Processing means which is configured to determine feedback information based on one or more of the inhalation characteristics of the user.

Preferably, the inhaler monitoring device further comprises feedback means which is configured to provide visual, audible and/or haptic feedback to the user based on the feedback information and/or one or more of the inhalation characteristics, preferably wherein the feedback means is configured to provide the feedback to the user in real time. Ideally, the feedback means comprises a plurality of LEDs which are located on the body of the device, which are configured to illuminate in a predetermined sequence based on the one or more inhalation characteristics of the user.

Preferably, the one or more sensors comprise: at least one air pressure sensor; at least one movement sensor and/or at least one environmental sensor.

Ideally, the feedback information comprises at the least the inhalation characteristics of the user.

Preferably, the feedback information comprises a user score which is determined based on the user's inhalation characteristics, preferably a separate user score is determined in respect of the each of the different inhalation characteristics of the user.

Ideally, the user score is determined based on the user's inhalation characteristics with respect to one or more pre-determined thresholds for the one or more inhalation characteristics.

Preferably, the processing means is configured to continuously monitor the user's inhalation characteristics over a period of time and alter the user score(s) based on one or more changes in the user's inhalation characteristics over the period of time.

Ideally, the processing means is configured to continuously monitor the user's inhalation characteristics over a period of time and alter the one or more pre-determined threshold values for the inhalation characteristics based on one or more changes in the user's inhalation characteristics over the period of time.

Preferably, the pre-determined thresholds for the one or more inhalation characteristics vary based on one or more user attributes such as age, medical condition(s), gender or any other suitable user attribute.

Ideally, the processing means is configured to apply an Al algorithm to the inhalation characteristics to determine the feedback information.

A second aspect of the present disclosure provides an inhaler apparatus comprising:
An inhaler configured to dispense medication;
A spacer; and
An inhaler monitoring device, the inhaler monitoring device comprising:
   A body comprising an inlet and an outlet which are in fluid communication with respect to one another to define a channel therebetween;
   Wherein the inhaler is removably coupled to the inlet and the spacer is removably coupled to the outlet through which a user can inhale the dispensed medication from the inhaler;
   one or more sensors which are configured to measure one or more inhalation characteristics when the user inhales the dispensed medication from the inhaler through the inhaler monitoring device via the spacer in-use;
   Processing means which is configured to determine feedback information based on one or more of the inhalation characteristics of the user.

Preferably, the inhaler monitoring device comprises the inhaler monitoring device of first aspect of the present disclosure.

A third aspect of the present disclosure provides an inhaler monitoring system, the inhaler monitoring system comprising:
The inhaler apparatus of the second aspect of the disclosure; and
A computing device;
Wherein the inhaler monitoring device is configured to transmit the feedback information to the computing device;
Wherein the computing device is configured to receive the feedback information and provide this to the user.

Ideally, wherein the computing device is configured to provide further user specific feedback to the user based at least on the feedback information received from the inhaler monitoring device.

Preferably, wherein the computing device comprise a personal computing device such as a smartphone, tablet, laptop, smartwatch or any other suitable personal computing device.

Ideally, wherein the feedback information comprises media data which is provided to the user by the computing device, preferably, wherein the media data comprises video, image and or audio media data.

Preferably, wherein the feedback information comprises a user score, ideally wherein a separate user score is determined for each of the one or more inhalation characteristics of the user, preferably, wherein the user score for each of the inhalation characteristics is dynamically weighted based on one or more of the user's inhalation characteristics. Ideally, the inhaler monitoring system further comprising a central server which is communicatively coupled to the computing device and/or inhaler monitoring device.

Preferably, wherein the central server is configured to consolidate the feedback information provided by the inhaler monitoring device and/or the user inhalation characteristics with supplemental data to determine an advisory action based on the consolidated user inhalation characteristics and supplemental data.

Ideally, wherein the supplemental data comprises further clinical or physiological data regarding the user, further data regarding the medication being received by the user and/or further environmental information regarding the location where the user made use of the inhaler apparatus and/or third party user data.

Preferably, wherein the advisory action comprises a non-adherence action and/or a risk action, preferably wherein a non-adherence action comprises wherein the central server is configured to communicate with the computing device to notify the user of one or more actions to take to improve their inhalation characteristics, optionally wherein a risk action comprises wherein the central server is configured to communicate with the computing device to notify the user of their risk of their medical condition deteriorating or improving based on their inhalation characteristics.

Ideally, wherein the central server is configured to contact the user's clinician or guardian based on their inhalation characteristics.

Preferably, wherein the computing device is configured to apply an Al algorithm to the received inhalation characteristics from the inhaler monitoring device to determine the user specific feedback; and/or wherein the Al algorithm is trained with the user's inhalation characteristics over a period of time and/or supplemental data received from the central server such that the user specific feedback provided to the user dynamically adapts over time; and /or wherein the central server is configured to apply an Al algorithm to, the user inhalation characteristics or the user inhalation characteristics and supplemental data, when the central server determines the advisory action.

Advantageously, over time, user individualised inhalation characterisation scores will allow the determination of risk of increased symptoms based on one or more aspects of the user's historical inhalation characteristics, local environmental conditions and other health status indicators (supplemental data)

Preferably, the inhaler monitoring system further comprises a central server which is communicatively coupled to the computing device and/or inhaler monitoring device.

Ideally, an Al algorithm (a 'training algorithm') embedded in the data capture device which is configured to use measured inhalation rates/scores to adapt the sensitivity of the measuring method to a 'relaxed measuring' or 'increased measuring' constraint threshold, when a user first begins to interact with the device. As a user gains confidence in using the inhaler device, and as correct inhalation technique is achieved (through continued use), the algorithm will adjust the measuring constraint in small steps to an 'ideal' target setting, with the aim of maintaining correct inhalation technique.

A further aspect of the present disclosure provides a method for monitoring inhaler technique competence, the method comprising:
Receiving one or more inhalation characteristics of a user;
Determining feedback information based on the one or more inhalation characteristics of the user; and
Providing the feedback information to the user;
Wherein the feedback information comprises a user score which is determined based on the user's inhalation characteristics.

Ideally, a separate user score is determined in respect of the each of the different inhalation characteristics of the user.

Preferably, the user score is determined based on the user's inhalation characteristics with respect to one or more pre-determined threshold values for the one or more inhalation characteristics.

Ideally, the method further comprising monitoring the user's inhalation characteristics over a period of time and/or number of inhaler uses and altering the user score(s) based on one or more changes in the user's inhalation characteristics over the period of time and/or number of inhaler uses.

Preferably, the method further comprising monitoring the user's inhalation characteristics over a period of time and/or number of inhaler uses and altering the one or more pre-determined threshold values for the inhalation characteristics based on one or more changes in the user's inhalation characteristics over the period of time and/or number of inhaler uses.

Ideally, the pre-determined threshold values for the one or more inhalation characteristics are varied based on one or more user attributes such as age, medical condition(s), gender or any other suitable user attribute.

Preferably, the method further comprises receiving one or more spirometry measurements of the user. Ideally wherein the spirometry measurements are provided to the user, typically incorporated within the feedback information.

Ideally the spirometry measurements comprise peak expiratory flow, forced vital capacity and/or forced expiratory volume.

A further aspect of the present disclosure provides a spirometry device comprising:
A body comprising an inlet and an outlet which are in fluid communication with respect to one another to define a channel therebetween;
Wherein the inlet is for coupling to a mouthpiece into which a user can exhale;
wherein the outlet is for coupling to a spacer such that the mouthpiece is in fluid communication with the spacer;
one or more sensors which are configured to measure exhalation data indicative of one or more spirometry measurements when the user exhales into the mouthpiece; and
Processing means which is configured to determine the one or more spirometry measurements feedback information based on the exhalation data.

Preferably, wherein the one or more spirometry measurements comprise peak expiratory flow, forced vital capacity and/or forced expiratory volume.

Ideally, wherein the spirometry device further comprises a feedback means which is configured to provide the one or more spirometry measurements to the user. Typically the feedback means comprise an external computing device, such as the user's smartphone, having a corresponding pre-installed application installed thereon which is configured to display the spirometry measurements to the user via a display of the external computing device.

A further aspect of the present disclosure provides a method for guiding optimal inhalation technique of a user using an inhaler monitoring device as defined in the first aspect of the disclosure, the method comprising:
Determining a threshold for each of a plurality of different steps of inhalation;
Measuring a user's inhalation characteristics;
Presenting a prompt to the user according to the determined threshold for each of the steps of inhalation in a pre-determined sequence;
Determining if the user has met the threshold for each of the steps of inhalation;
wherein each of the prompts corresponding to each of the different steps of inhalation are provided, typically displayed, to the user until the determined threshold for that step is met prior to providing the subsequent prompt for the next step in the pre-determined sequence.

Preferably, the different steps of inhalation comprise: shake duration, shake to dispense interval, dispense to inhale time, inspiratory flow rate and volume inhaled.

Ideally, the prompts comprise a visual prompt presented on an external computing device such as a smartphone of the user typically having a pre-installed application thereon.

Preferably, the user's smartphone is communicatively coupled to the inhaler monitoring device such that determining if the user has met the threshold for each of the steps of inhalation is determined based on data acquired by the one or more sensors of the inhaler monitoring device, such as the pressure sensor and/or movement sensor, which is transmitted in real time to the user's smartphone, and ideally presented to the user via a Graphical User Interface (GUI), such that the user is guided through each inhalation step, with one or more respective prompts, based on data provided by the inhaler monitoring device. Advantageously, the method automates the presentation of the prompts to the user in accordance with the steps of inhalation and ideally the thresholds determined accordingly for each.

Advantageously, the external computing device, in particular the GUI shown thereon, is configured to automate the presentation of audio-visual cues presented on the external computing device in a manner which demonstrates to users the correct sequence and/or timing of events for optimised inhalation of medication via an MDI using the inhaler monitoring device.

These and other objects, advantages, purposes and features of the present invention will become apparent upon review of the following specification in conjunction with the drawings.

### Brief Description of the Drawings

The invention will now be described with reference to the accompanying drawings by way of example only, within which:
**Figure 1** is a front perspective view of an inhaler monitoring device;
**Figure 2** is a rear perspective view of the inhaler monitoring device
**Figure 3** is a rear perspective view showing the inhaler monitoring device, a spacer and an inhaler, which when coupled provide an inhaler apparatus;
**Figure 4** is a front perspective view of the inhaler apparatus;
**Figure 5** is a side perspective view of the inhaler apparatus showing the various types of inhalers which may be coupled to the inhaler monitoring device;

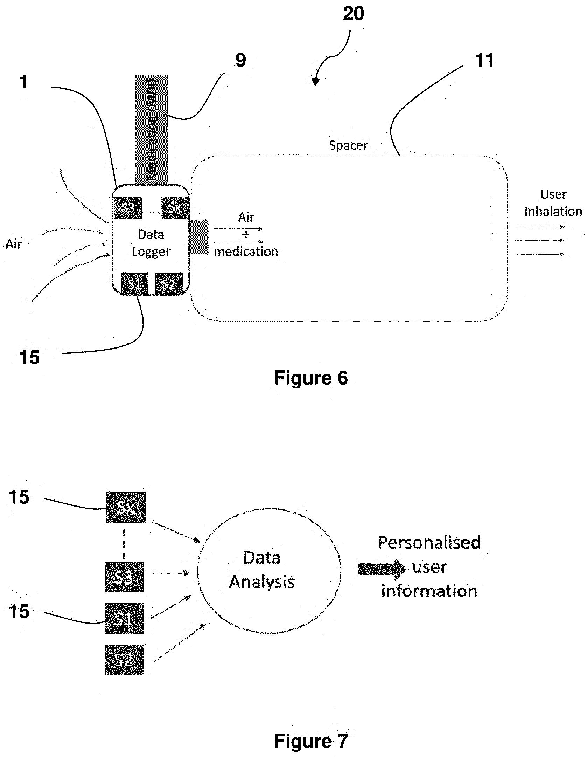

**Figure 6** is a diagram illustrating the inhaler apparatus;
**Figure 7** is a diagram illustrating the data acquired by the one or more sensors of the inhaler monitoring device;
**Figure 8** is a diagram illustrating the device architecture of the inhaler monitoring device;
**Figure 9** is a diagram showing an inhaler monitoring system embodying one aspect of the present disclosure;
**Figure 10** is a diagram illustrating how the rate of inhalation is calculated;
**Figure 11** is a diagram illustrating how the volume of inhalation is calculated;
**Figure 12** a further diagram illustrating how the volume of inhalation is calculated;
**Figure 13** is a side view of the inhaler monitoring device configured to obtain spirometry measurements in accordance with a further aspect of the disclosure;
**Figure 14** is a flow diagram illustrating an inhalation algorithm;
**Figure 15** is a diagram illustrating five steps of optimal inhalation technique and the associated time for each of the steps; and
**Figure 16** is a sequence of diagrams showing prompts provided to the user via a graphical user interface (GUI) embodying a method for guiding optimal inhalation technique of a user using an inhaler monitoring device.

### Detailed Description

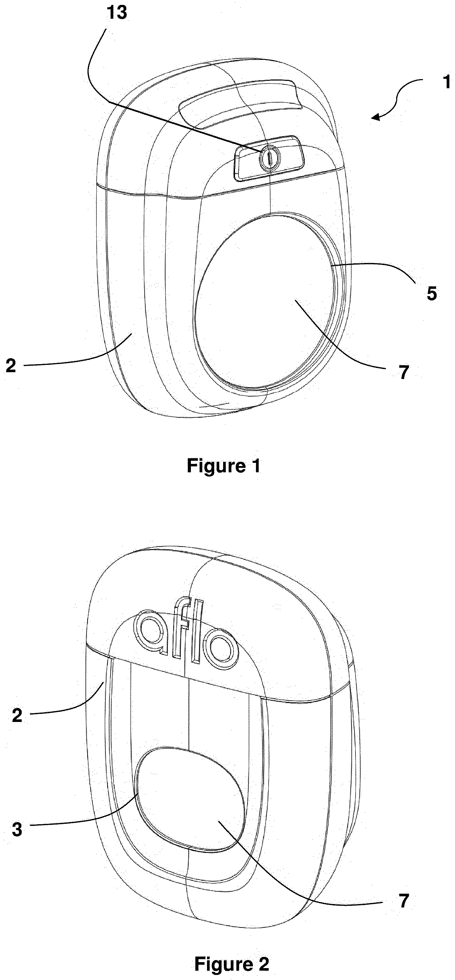

Referring now to the drawings, in particular Figures 1 and 2 there is shown, generally indicated by the reference numeral 1, an inhaler monitoring device. The inhaler monitoring device 1 comprises a body 2, typically substantially cuboidal in shape, comprising an inlet 3 and an outlet 5 which are in fluid communication with respect to one another to define a channel 7 therebetween. The inlet 3 comprises a first opening 3 and the outlet comprises a second opening 5. The first and second openings 3, 5 are typically provided on opposing sides of the body 2 and are aligned with respect to one another such as to define the channel which extends through the body 2 of the inhaler monitoring device 1.

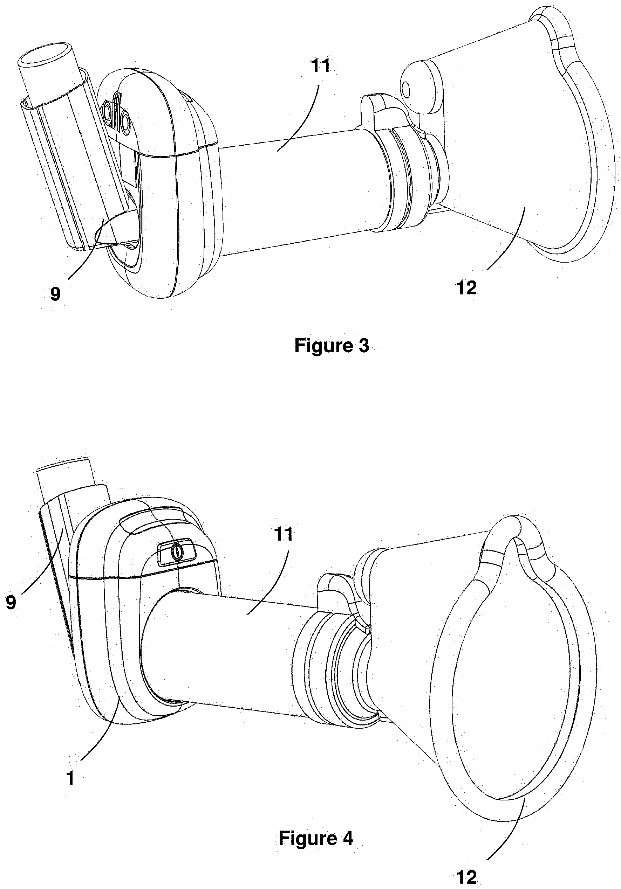

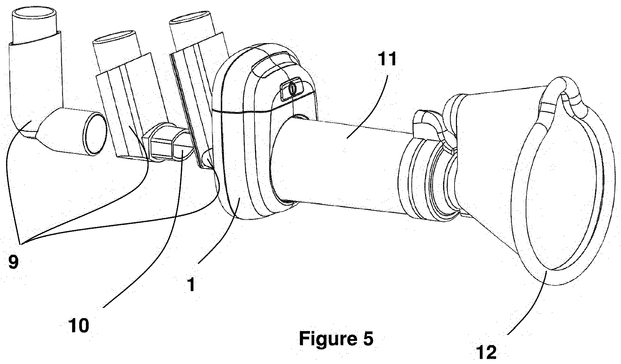

The inlet 3 is for removably coupling to an inhaler 9 (such as is shown in Figures 3 to 5) which is suitable for dispensing a medication. To this end, the inlet 3 is shaped and dimensioned to receive and retain at least a part of the inhaler 9, typically a mouth piece portion 10, via a friction fit coupling. The inlet 3 may comprise a flange, which is typically made from a resiliently deformable material such as rubber or any other suitable resiliently deformable material, for aiding in receiving and retaining the inhaler 9 therein. Alternatively, the body 2 and the inhaler 9 may comprise corresponding male and female parts for coupling the inhaler 9 to the inhaler monitoring device 1. The inhaler 9 typically comprises a Metered Dose Inhaler (MDI) commonly used for the treatment and management of respiratory diseases. An MDI is designed to deliver therapeutic agents, e.g. medicaments, to the human respiratory tract. Accordingly, the MDI contains the active substance, dissolved or suspended, in a fluid propellant system that contains at least one liquefied gas in a pressurized container that is sealed with a metering valve. The actuation of the valve delivers a metered dose of medicament in the form of an aerosol spray and is directed by a suitable adapter/activator for dispensation via oral inhalation. It should be understood that references to "an inhaler" throughout the specification are intended to mean a MDI as described herein. The appearance of inhalers can vary based on the manufacturer however this is largely a difference of aesthetics, the operating principle remains the same regardless, Figure 5 shows some example variations of inhalers 9 each of which can be used with the inhaler monitoring device 1 of the present disclosure.

The outlet 5 is for removably coupling to a spacer 11 (such as is shown in Figure 3) having a mouthpiece 12 through which a user can inhale the dispensed medication from the inhaler 9. The spacer, also known as a valved holding chamber, is a well understood device in this field which typically comprises an elongate tube having the mouthpiece 12 provided for a user at one end and means for coupling to a MDI at the opposing other end. In the present case, the end of the spacer 11 which would usually be coupled directly to an MDI, is instead coupled to the outlet 5 of the inhaler monitoring device 1. To this end, the outlet 5 is shaped and dimensioned to receive and retain at least a part of the spacer 11, typically the end opposing the mouth piece portion 12, via a friction coupling, the outlet 5 typically having a diameter slightly larger than the end of the spacer 11. The outlet 5 may comprise a flange, typically a rubber flange or the like, for aiding in receiving and retaining the spacer 11 therein. Alternatively, the body 2 and the spacer 11 may comprise corresponding male and female parts for coupling the spacer 11 to the inhaler monitoring device 1.The inhaler monitoring device 1 when coupled to both the inhaler 9 and spacer 11 defines an inhalation apparatus 20 embodying an aspect of the present disclosure.

The inhaler monitoring device 1 further comprises one or more sensors 15 which are configured to detect and/or measure one or more inhalation characteristics when the inhaler 9 is actuated to dispense medication and the user inhales the dispensed medication from the inhaler 9 through the inhaler monitoring device 1 via the spacer 11 in-use. For example, the one or more inhalation characteristics may comprise: air pressure, both internal and/or external to the inhaler monitoring device 1; movement of the inhaler monitoring device 1 and/or any other suitable characteristics. To this end the one or more sensors may comprise a pressure sensor for measuring air pressure and/or a movement sensor such as an accelerometer or the like for detecting and measuring movement such as shaking of the inhaler monitoring device 1.

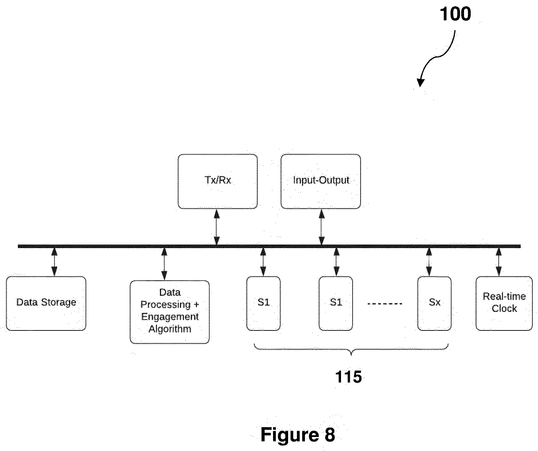

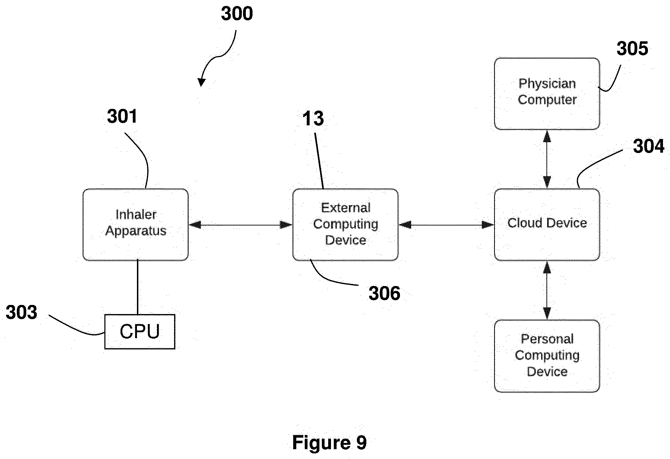

The inhaler monitoring device 1 also comprises processing means, such as a CPU or the like (which is shown at Figures 8 and 9 of the accompanying drawings), which is configured to generate feedback information based on the data indicative of the one or more inhalation characteristics received from the one or more sensors 15. The feedback information may comprise the inhalation characteristics and/or further information derived based on one or more of the inhalation characteristics. The processing means typically comprises one or more microcontrollers which are located within the body 2 of the inhaler monitoring device 1, alternatively the processing means may include any suitable processing means. The inhaler monitoring device 1 may further comprise one or more feedback means (not shown) which is configured to provide visual, audible and/or haptic feedback to the user based on the determined feedback comprising the measured inhalation characteristic or combination of characteristics. For example, the feedback means may comprise a plurality of lights located upon the body 2 such as that shown in Figures 1 and 2, a certain number of which will adopt an illuminated state corresponding to a specific measured characteristic or combination of characteristics. The feedback means may also be configured to provide or indicate diagnostic feedback regarding the current operating status of the inhaler monitoring device 1. Additionally or alternatively the feedback means may comprise: a speaker through which audible feedback may be provided to the user; a vibratory motor through which haptic feedback may be provided to the user; and/or a display which is configured to provide further visual feedback to the user. The inhaler monitoring device 1 preferably further comprises wired and/or wireless transmission means such that the data obtained by the one or more sensors of the inhaler monitoring device 1 and/or the inhalation characteristics and/or the feedback information determined by the processing means may be transmitted to an external and/or remote computing device or the like for further analysis and/or the provision of feedback to the user. The wireless transmission means typically comprises a lower power wireless transmission means such as Bluetooth ^{®}, however it may additionally or alternatively comprise Wi-Fi ^{®}, NFC or any other suitable wireless transmission means.

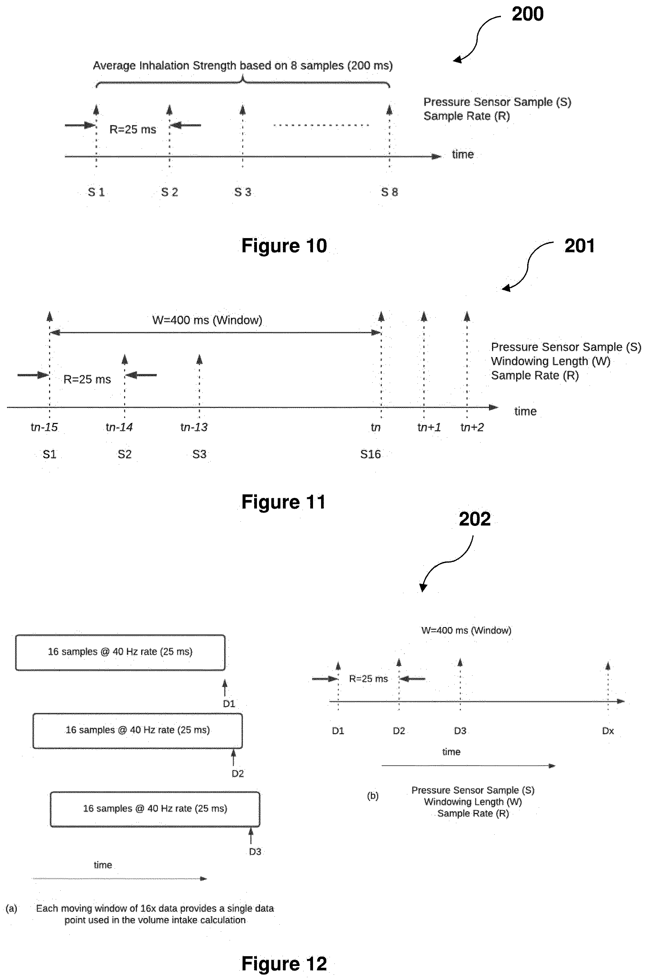

Ideally, a heuristic method may be used to calculate the airflow rate and volume inhaled by the user. Preferably, to calculate the rate of inhalation, 8 samples of sensor are typically read every 25ms, as shown in Fig. 10, and converted to a ml/sec rate values. The average across the typical 8 samples is then calculated to provide a inhalation rate value which is matched against the bands obtained via empirical measurements. The matching band colour is then illuminated on the device.

### Calculating the Volume of Inhalation

To calculate the volume of air inhaled, a moving average of inhalation rate is first established for each sample point (typically 40Hz rate, 25 ms). This is due to the dynamic nature of the sensor output (note: it is dynamic but has a low standard deviation). Each moving window typically consists of 16 data points (S1 to S16) which are typically read at every time t=25ms. The initial inhalation rate is not calculated until the first 16 samples have been read. Fig 11 illustrates the windowing of the sensor data over the example 400 msecs period where each sensor sample (S) is firstly converted using eqn. (1). The converted samples S1 to S16 are then averaged to provide a single value for the sample point at time t*ₙ*. The next sample point at time t*ₙ₊₁*, the window moves forward and is based on the new sample point plus the previous 15 sample points. This process repeats as shown in Fig 12 (which shows moving average using the example 400msec window). where each window outputs a data point D (windowed average inhalation rate) for every time interval of 25ms. $Normalised _ Sensor _ Data = \left(\left(Sensor_raw_data/24\right)+165\right)$ Eqe. (1)

The total volume of inhaled air is calculated using equation (2) where each inhalation rate, *D,* is accumulated over the time of inhalation. The principle is that *D* inhalation rate is held for 25ms and therefore over time the total volume of inhaled air can be calculated. $Total volume intake \left(ml\right) = {\sum }_{i = 1} Di / 40$ Eqe. (2)

Each inhalation rate is typically recorded every 25 msec therefore has 1/40 of contribution to the total. The accumulation process using eqn 2 continues for all data points D until the example max volume (375ml) has been met.

In an alternative embodiment of the disclosure, the inhaler monitoring device 1 may comprise at least two sensors, preferably comprising at least two air pressure sensors. A first air pressure sensor (not shown) which is configured to detect and measurement the air pressure internal to the inhaler monitoring device 1, typically within the channel 7 and further preferably being located closer to the outlet 5 than the inlet 3. A second air pressure sensor (not shown) which is configured to detect and measure air pressure external to the inhaler monitoring device 1, i.e. the air surrounding the device 1. The second air pressure sensor may be located close to the inlet 3 of the inhaler monitoring device, for example below the inlet 3 of the inhaler monitoring device 1 such that the sensor remains exposed when the inhaler 9 is coupled to the inlet 3.

In-use when the inhaler 9 is actuated, typically by the user or an accompanying person or medical professional, the medication (dosage) is released from the inhaler 9 into the spacer 11 via the inhaler monitoring device 1. The user inhales via the mouthpiece 12 of the spacer 11 breathing in a mixture of air and the dispensed medication from the inhaler 9. Air enters the spacer 11 from the inhaler 9 via the inhaler monitoring device 1, the first air pressure sensor (not shown) is configured to measure the air pressure within the channel 7 as the user inhales the medication, to this end the first air pressure sensor is typically located within close proximity to the spacer 11. At substantially the same time or within close proximity thereto, the second air pressure sensor is configured to measure the air pressure external to the inhaler monitoring device 1. The measuring of the air pressure external to the inhaler monitoring device provides a reference value which the air pressure measured by the first air pressure sensor may be compared to in-use. The air pressure measurements obtained by the first and second air pressure sensors are provided to the processing means incorporated within the inhaler monitoring device, however additionally or alternatively they may also be transmitted to an external computing device (not shown) remote to the inhaler monitoring device 1. The difference between the air pressure measurements from the first and second air pressure sensors is used by the processing means to determine airflow through the inhaler monitoring device 1 and typically therefore compliance for volume ranges. Further the detection of a rapid pressure difference between the first and second sensors provides an indication that medication has been dispensed from the inhaler 9.

The inhaler monitoring device 1 may further comprise one or more movement sensors (not shown) which are configured to detect movement, in particular shaking or other physical agitation, of the inhaler monitoring device 1 and correspondingly therefore the spacer 11, inhaler 9 which are coupled thereto. The movement sensor may comprise an accelerometer or any other suitable sensor for measuring movement. The movement information acquired by the movement sensor is provided to the processing means. The inhaler monitoring device 1 further typically comprises an on/off button to vary the device between respective on and off states. Additionally or alternatively the inhaler monitoring device may use one or more of sensors to determine when to adopt an on or off state. For example when the movement sensor detects movement the inhaler monitoring device 1 may adopt an on state, wherein if the movement sensor fails to detect movement for a pre-set amount of time the inhaler monitoring device 1 may be configured to adopt an off state etc.

The inhaler monitoring device 1 may also comprise one or more environmental sensors (not shown) which are configured to monitor one or more environmental conditions within the immediate environment of the inhaler monitoring device 1 in-use. The one or more environmental sensors may be configured to monitor the environmental conditions prior to, during and/or after the dispensation of medication from the inhaler 9 through the inhaler monitoring device 1, spacer 11 to the user. Additionally or alternatively, the environmental sensor may be configured to acquire data regarding the environmental conditions surrounding the inhaler monitoring device 1 at pre-determined intervals. The one or more environmental conditions may include: temperature, humidity, ozone, particulates (dust, dander PM2.5, PM10), pollen, spores and bacteria and/or any other suitable environmental parameter. The incorporation of one or more environmental sensors generates a richer real time dataset to understand the full impact of the surrounding environmental conditions on short and long-term use of the MDI and the user's medication management plan. The environmental information provides further data to the processing means for assessing the local conditions when the user takes their medication thereby and provides further information to the user about the impact air quality has on their inhalation characteristics. This provides enhanced direction on the type of feedback that can be communicated to a user to best advise on how to manage their respiratory condition: e.g. open windows, remove pollutant sources such as open fires, reduce animal dander sources, refrain from smoking indoors, etc.

Based on the data provided by the one or more sensors of the inhaler monitoring device 1 including for example, the first and second air pressure sensors and/or the movement sensor and/or the environmental sensors, the processing means is configured to determine and/or acquire one or more inhalation characteristics when the user inhales the dispensed medication from the inhaler 9 through the inhaler monitoring device 1 via the spacer 11 in-use. The one or more inhalation characteristics may include: airflow characteristics; intensity and duration of movement of the inhalation monitoring device 1, prior to, during and/or after the dispensation of medication from the inhaler 9; the time at which medication was dispensed; the time between dispensation of medication and inhalation; inhalation rate; medication dose and/or any other suitable characteristic of inhalation. The inhalation characteristics are typically recorded locally on a memory communicatively coupled to the processing means; however it may additionally or alternatively be transmitted remotely from the inhaler monitoring device 1 using wired or wireless transmission means to an external memory location or external computing device.

Preferably, the inhalation characteristics are stored locally on the inhaler monitoring device 1, optionally the inhalation characteristics may be transmitted using wireless transmission means to a personal computing device such as a user's smartphone, smartwatch, tablet laptop or desktop computer or any other suitable display means such that the user can view the inhalation characteristics and/or feedback information determined based on their inhalation characteristics on their personal computing device. The processing means is configured to determine the feedback information in response to the inhalation characteristics, wherein the feedback information may comprise the individual inhalation characteristics as well as further information derived based on the one or more inhalation characteristics. The feedback information may additionally or alternatively be provided to the user via the feedback means. The feedback means which as mentioned previously and as shown in Figures 1 and 2 may comprise the plurality of lights, typically LEDs, located on the body of the inhaler monitoring device 1. The feedback means may also be configured to provide or indicate diagnostic feedback regarding the current operating status of the inhaler monitoring device 1 such as but not limited to: the operating state of the inhaler monitoring device 1 e.g. on/off; the battery level; whether or not one or more inhalation characteristics have been successfully determined; whether or not the feedback information and/or the inhalation characteristics have been successfully transmitted to a remote computing device such as the user's personal smartphone or the like or any other suitable operating characteristic. The feedback means 13 may also be configured to provide feedback information regarding the environmental conditions acquired by the environmental sensors pre, during and post dispensation of medication from the inhaler 9.

The feedback means 13 may be configured to provide feedback to the user not only after inhalation of the dispensation of the medication from the inhaler 9 but also prior to and/or during the dispensation of the medication from the inhaler 9 in real time. For example, prior to the dispensation of the medication inhalers typically require a certain amount of agitation by shaking to mix the propellant and medication stored therein, the feedback means 13 may be configured to indicate to the user, using measurements obtained by the one or more sensors of the inhaler monitoring device 1, typically the movement sensor thereof, when the inhaler monitoring device 1 and the coupled inhaler 9 have been sufficiently agitated for ideal medication dispensation from the inhaler 9.

Once the inhaler 9 is actuated to dispense medication, with the user inhaling through the spacer, the feedback means 13 may be configured to indicate to the user the duration of time for which they should continue to inhale, this being determined based on the spacer type and measurements obtained by the one or more sensors, typically the air pressure sensors, in combination with the processing means. For example, where the feedback means 13 comprise a plurality of LEDs located on the body of the inhaler monitoring means 1, the lights may illuminate in an ascending sequence corresponding to the length of time the user should continue to inhale, for example if there are several LEDs the first would illuminate after one second, the second LED after additional time etc. up to the point where when the LEDs are illuminated the user should stop inhaling. Additionally or alternatively, the LEDs may illuminate in a predetermined sequence to indicate that the user should increase their inhalation rate based on the airflow determined to be passing through the inhaler monitoring device 1.

It should be understood that, as mentioned previously, the feedback means 13 is not limited to visual, audio and/or haptic feedback means provided on the inhaler monitoring device itself as in in addition to or alternative to this, the feedback means 13 may comprise an external computing device and/or a remote computing device such as a cloud based server which is configured to receive the feedback information from the inhaler monitoring device 1 and provide user specific feedback to the user based on the feedback information, the feedback information including at least the user's inhalation characteristics. This feedback may be in the form of visual or audible feedback and typically comprises data presented in graphed or tabular scores or media data such as a video or sound file which is specifically tailored to the user's determined inhalation characteristics. For example, where the inhalation characteristics indicate that the user did not inhale for a sufficient period of time the media data provided to the user may comprise a score presented via an external computing device and a video file or other means of guidance demonstrating the optimum time period to inhale. Similarly for example, where the inhalation characteristics indicate that the inhaler monitoring device 1 and coupled inhaler 9 were not shaken sufficiently or for a long enough period of time prior to medication dispensation, the media data provided to the user may comprise a score presented via an external computing device and a video file showing the optimum technique for shaking the inhaler monitoring device 1 in-use.

A diagram showing the typical device architecture of the inhaler monitoring device 1, 100 embodying the first aspect of the disclosure is shown at Figure 8. The data processing device architecture shown in Fig. 8 includes several sensors 115, real-time clock (RTC), data processor 303, data storage, ports for flexible TX-RX and standard Input-Output. Several sensors enable the capture of bespoke data on agitation and inhalation rates as examples. The RTC allow timestamping of device uses and can be used as a reference timer. The TX-RX port can be a wireless communication port for sending and receiving data and the Input-Output port can represent, for example, switches (inputs) or LEDs (outputs). These ports are termed the *User-Interface* as any interaction between the device and user, via another computing device, is achieved using these two ports. The data processor contains key computing elements and can be viewed as microcontroller which can process both the engagement algorithm and/or any data from the user-interface.

A further embodiment of the present disclosure provides spirometry functionality to the inhaler monitoring device 1 such that it is configured to perform as a spirometry device, spirometry measurements comprising peak expiratory flow, forced vital capacity, forced expiratory volume. A peak flow meter is a handheld device that allows individuals with respiratory diseases to measure how well their lungs can expel air. The peak flow meter is used by blowing a rapid blast of air through a mouthpiece and the peak flow meter then measures the flow rate in litres per minute. Peak expiratory flow (PEF) is a simple measurement of how quickly a person can blow air out of their lungs and is used to help diagnose and monitor patients with asthma. It measures a person's maximum speed of expiration, as measured with the peak flow meter which is used to monitor a person's ability to breathe out air as mentioned previously. The test involves a patient blowing at a maximum rate into the peak flow meter and their flow rate score indicates whether the patient airways are narrowed (degree of obstruction) which is indicative of asthma, typically confirmation of this diagnosis is also needed using a spirometry test. In patients with asthma, the PEF value correlates reasonably well with the percent predicted value for the forced expiratory volume in one second (FEV 1) and provides an objective measure of airflow limitation when spirometry is not available. PEF is routinely used in clinical care and respiratory symptom assessment although the evidence that peak flow readings are related to symptoms is moderate.

It has been established that consistent measurement of PEF allows a sufferer to monitor their condition where the PEF score is indicative of improvement or deterioration. Using a peak expiratory flow meter can help a patient to track their control of asthma, indicating how well prescribed treatment is working and to help recognise signs of exacerbation. Clinical advice is that PEF measurements should be carried out regularly so that patients can self-assess their condition. Additionally, patients may be required by their doctor to maintain a PEF diary for a predefined period of time to help confirm a diagnosis.

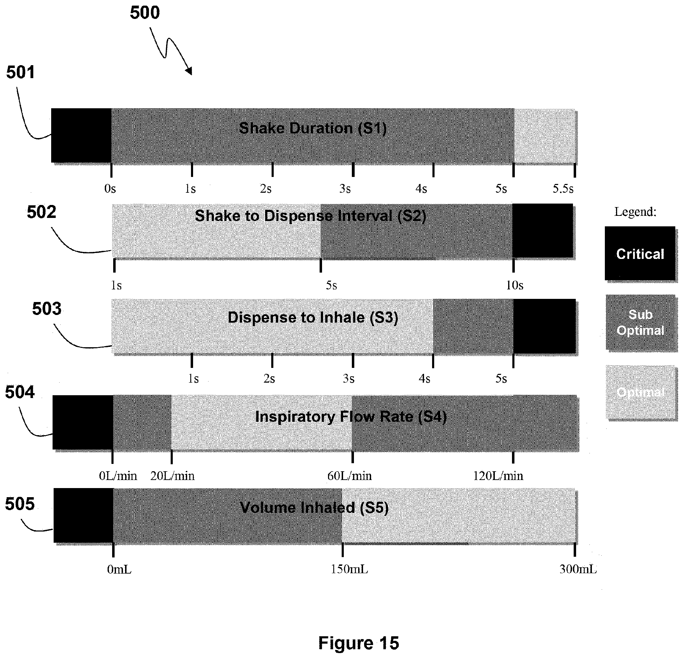

Accordingly this embodiment, which is complimentary to the previous defined embodiments, comprises reconfiguring the inhaler monitoring device 1, either prior to or subsequent to the use of the metered dose inhaler 9, or in isolation with respect thereto, to perform the function of spirometry. The spirometry data acquired by the inhaler monitoring device 1 and the spirometry measurements determined therefrom may subsequently be provided to the user to inform the user of the current status of their condition and in particular to any changes in their condition. The inclusion of spirometry functionality within the existing inhaler monitoring device 1 (also known as the aflo^{™} device) permits sufferers to continually monitor their condition. The inhaler monitoring device 1 currently allows asthma and COPD patients to continually monitor the five steps of inhalation, shown for example at Figure 15, thereby facilitating self-management of their condition. The inclusion of regular spirometry evaluation would allow better correlation between their condition status and the five steps of inhalation. Spirometry measurements can also be used to correlate with an exposure to a possible asthma trigger, such as allergies or environmental changes (e.g. household dust, animal dander, cold weather, air pollution) or other events that may cause deterioration in a patient symptom control. Relating peak flow to the steps of inhalation as well as environmental changes improves the accuracy and timing of prompts to the user and the respiratory consultant thereby enhancing the quality of interventions.

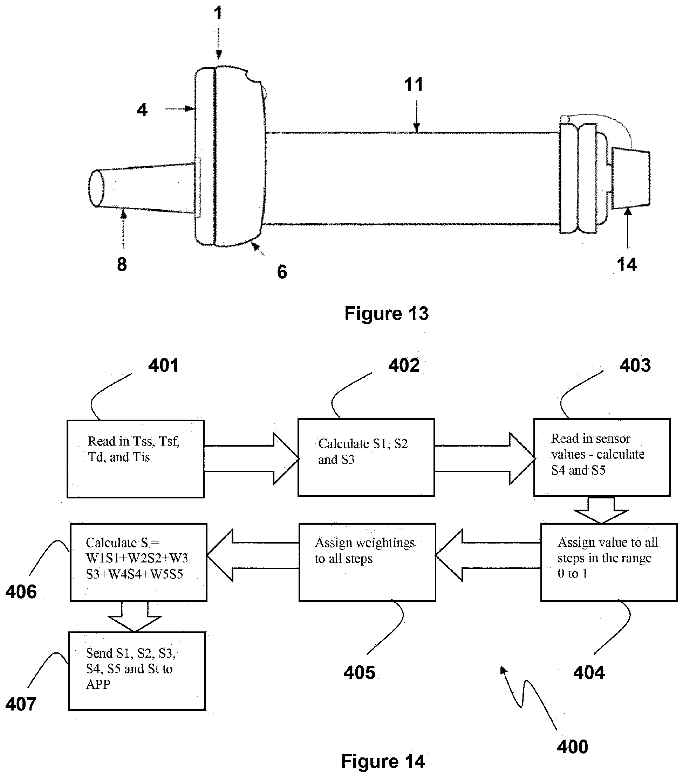

The spirometry functionality embodiment where the inhaler monitoring device 1 is configured to acquire and or determine spirometry measurements of a user is shown in Figure 13 generally indicated by the reference numeral 150. The inhaler monitoring device 150 is identical to that previously described in relation to Figures 1 and 2 having identical features to that previously described. Further to this, the inhaler monitoring device 150 comprises a mouthpiece 8 which is coupled thereto, in particular to the inlet 3 located on a first side 4 thereof. The spacer 11 is ideally coupled to the outlet 5 located on a second side 6 of the inhaler monitoring device. Further typically the opposing end of the space 11, i.e. the end furthest from that which is coupled to the inhaler monitoring device 1, may comprise a cap 14 which is removably coupled to the spacer 11, which may be configured to limit and/or restrict the airflow there through, at least in part, in-use.

As mentioned, spirometry measurement would be accommodated using the mouthpiece 8 which is removably coupleable to the inlet 3 at the first side 4 of the inhaler monitoring device 1. The inhaler monitoring device 150 is configured to acquire spirometry data using the one or more sensors 15, typically comprising one or more pressure sensors, when the user exhales or blows into the inhaler monitoring device 150 via the mouthpiece 8 as described and shown in figure 13. Preferably spirometry data would be captured using the existing pressure sensor(s) within the inhaler monitoring device 150 over at least three exhalations, in line with clinical best practice guidance, and the data being presented immediately to the user such as via the accompanying app or other suitable computing means.

For example, in-use to provide spirometry functionality to the inhaler monitoring device 1 the user would couple the mouthpiece 8 to the inhaler monitoring device 1, in particular to the inlet 3 located on the first side 4 thereof. If the user is obtaining spirometry data subsequent to inhalation characteristics as described in previous embodiments, for example at pages 7 to 14, then the user may be required to detach the coupled inhaler 9 and replace this with the mouthpiece 8. The spacer 11 is ideally coupled to the outlet 5, located on the second side 6, of the inhaler monitoring device 1 such as is typically also the case for when the inhaler monitoring device 1 is being used to obtain inhalation characteristics as detailed previously. The device is configured to obtain the user's exhalation data when they exhale, typically for at least three exhalations, into the device 150 via the mouthpiece 8. The inhaler monitoring device 150 as mentioned previously typically includes processing means 303 which is configured to generate feedback information based on the data received from the one or more sensors 15. Alternatively, the inhaler monitoring device 150 may comprise a transmitter/transceiver which is configured to transmit the spirometry data measurements, acquired by the sensors of the device 150, to an external computing device 306 for processing the data to generate feedback information. To this end the feedback information may comprise the spirometry data itself and/or further information derived based on the spirometry measurements. In particular the processing means is configured to determine spirometry measurements including one more of peak expiratory flow, forced vital capacity, forced expiratory volume for the user based on the spirometry data acquired by the inhaler monitoring device 150. The spirometry measurements may be used to determine how the user's spirometry measurements correspond to a predetermined measurement criteria; and/or how the user's spirometry measurements corresponds to other users with the same medical condition and/or the same age and/or gender etc. The user may be required to acquire their spirometry measurements at regular intervals and/or when a certain period of time ha elapsed since a previous measurement was acquired and/or as otherwise instructed by the complimentary software as detailed further herein.

The spirometry measurements acquired by the inhaler monitoring apparatus 1 may be provided to the user by the feedback means 13 or more preferably via the external computing device 306, wherein the inhaler monitoring apparatus 1 is configured to transmit the spirometry measurements and/or data via the wired and/or wireless transmission means to the computing device 306 which may be configured to visually display the spirometry measurements. For example, the computing device 306 may comprise the aflo^{™} respiratory management software, typically in the form of an app, pre-installed thereon, which is configured to quickly and easily exchange data with the inhaler monitoring apparatus 1 (aflo^{™} device) in real time and display this information to the user as mentioned previously in relation to earlier embodiments. In addition spirometry measurements and data will be used by the aflo^{™} software, comprising a data analytics engine to inform the user of changes in their condition in relation to one or more pre-defined criteria and/or thresholds such as the five steps of inhalation and/or other supplementary data. Real time and longitudinal feedback will be provided to the user via a data summary on the app and timely user/ clinician prompts/alerts.

### Engagement Algorithm

Analysis of sensor and related data captured by the Al algorithm embedded within the data capture device, in particular the inhaler monitoring device 1 and the inhaler monitoring device configured for spirometry functionality 150, will provide personalized information that can engage, motivate and steer a user towards optimal prescribed regiment and inhalation technique compliance.

The algorithm embedded in the data capture device, uses Al to provide a novel function that promotes better engagement with their medication regime to improve inhalation technique by dynamically adapting the sensitivity of the inhalation score to a user's initial technique. For novice users the algorithm will initially use a lower starting threshold for correct inhalation technique so engagement persists, with positive (inflated) feedback on inhalation scoring at the early stage. As the user's technique improves the algorithm dynamically moves towards reflecting the true threshold for inhalation technique competence. For example, as the user's inhalation technique competence improves over time, the threshold will be incremented by a step-value. Prior to this increment, the previous inhalation scores over a period of uses will be assessed. If the trend in the inhalation scores does not show an improvement across several uses, then the engagement algorithm will pause its incrementing process for a period of time so as to not discourage the user and afford them more practice at a lower threshold rate. If they do not improve, the engagement algorithm will proceed to increase the threshold at a slower rate. This has the effect of slowing the rate at which the algorithm moves towards the true threshold for inhalation competence rate. The reason for adopting this novel approach is to facilitate a 'relaxed measuring constraint' for novice users while at the same time providing the capability to dynamically adapt to challenge experienced users.

As mentioned, the processing means incorporated within the inhaler monitoring device 1 is further configured to continuously record the user's inhalation characteristics. The processing means is also configured to provide spirometry measurements over time and, to optimise the operation of the inhaler monitoring device 1 and the feedback provided thereby based on the changes determined in the inhalation characteristics or spirometry measurements over time. Further optionally this may also be through interactions with the external computing device and/or cloud based server. For example the processing means typically comprises an Al and/or machine learning algorithm which is configured to train itself using at least typical and non-typical user's inhalation characteristics over a period of time to optimise the operation of the inhaler monitoring device and the feedback provided thereby. To this end, the processing means is typically configured to determine inhalation scores based on at least the inhalation characteristics of the user however it may additionally be supplemented with additional data. Further the processing means is typically configured to determine spirometry measurements such as peak expiratory flow, forced vital capacity, forced expiratory volume based on the data acquired by the sensors 15. The spirometry measurements are typically presented to the user in the form of visual or audible feedback. For example this may be presented to the user as numerical values or it may be presented in graphed or tabular format or in the form of media data such as a video or sound file which is individually tailored to the user's determined spirometry measurements. Additionally or alternatively the spirometry measurements may also be provided to the user in the form of a score or scores based on, at least one of, or each of, peak expiratory flow, forced vital capacity, forced expiratory volume however it may additionally be supplemented with additional data. The spirometry measurements are typically provided as part of the feedback information using the feedback means 13 to the user. The Al algorithm may comprise an artificial neural network algorithm, a regression algorithm, a logistical model tree algorithm, a random forest algorithm, a fuzzy classifier algorithm, a decision tree algorithm, a hierarchical clustering algorithm, support vector machines, a k-means algorithm, a fuzzy clustering algorithm, a deep Boltzmann machine learning algorithm, a deep convolutional neural network algorithm, a deep recurrent neural network, or any combination thereof.

The individual inhalation scores provide an indicator of user competence at one or more steps of the inhalation such that the scores perform as training scores. Preferably, the individual scores for each inhalation characteristic provides an indicator of user competence across all steps of the inhalation technique. The inhalation technique comprises the one or more inhalation characteristics as mentioned previously, including at least: user engagement e.g. activation of the inhaler monitoring device 1; Inhaler agitation (shake) duration; time from shake 9 to dispense medication; the time taken from dispense to inhale commencement; the rate of inhalation of the user; and/or the volume inhaled by the user. Ideally, the user has to perform each of these inhalation steps efficiently to achieve optimum inhalation of the medication from the inhaler 9. Typically, the individual scores determined for each inhalation characteristic and/or spirometry measurement are provided visually to the user via the feedback means 13 and/or an external computing device and/or cloud based server (not shown). The individual scores for each inhalation characteristic can be used to coach the user to achieve correct inhalation technique, thus advantageously optimising their inhalation technique over time.

The processing means, typically located on the inhaler monitoring device 1 however it may additionally or alternatively comprise the external computing device 306 and/or cloud device 304, typically comprising the incorporated algorithm such as is shown in Figure 15, is configured to assign a score to each of the inhalation characteristics measured by the one or more sensors 15. These scores may be determined based on a pre-determined alphanumerical range, typically for example each measurement or characteristic will be scored in a range from 1 to 10 however it should be understood that this it not intended to be limiting and the range could comprise any suitable range. Additionally or alternatively, the inhalation characteristics may be transmitted from the inhaler monitoring device 1 to an external computing device 306 and/or cloud computing device 304 for determining the feedback information.

The processing means 303, 306, 304 may be configured to dynamically adapt the inhalation thresholds such that the inhalation range can be broadened for new users and narrowed for experienced users. An algorithm (the 'training algorithm') which is embedded in the inhaler monitoring device 1 or computing device or cloud based server or other, achieves the above using Al techniques to provide a method of adapting the sensitivity of the measuring method to a 'relaxed measuring constraint' when initially used. The 'relaxed measuring constraint' means that new users may initially get a mid-range inhalation score so as to not be discouraged with low scores (feedback on use of the device) during the training phase. As the user gains confidence in using the inhaler device, and correct inhalation technique is achieved (through continued use), the algorithm will start to adjust the measuring constraint in small steps to an 'ideal' target setting. Advantageously, this approach continually motivates the user to maintain and improve their inhalation technique.

The feedback information typically comprises at the least the inhalation characteristics of the user and/or one or more of the spirometry measurements. The feedback information comprises a user score which is determined based on the user's inhalation characteristics, preferably a separate user score is determined in respect of the each of the different inhalation characteristics of the user. A further score may optionally be determined for each of the spirometry measurements. Ideally, the user score is determined based on the user's inhalation characteristics with respect to one or more pre-determined threshold values for the one or more inhalation characteristics. Similarly the spirometry measurement scores may be determined based on comparing the user's spirometry measurements with respect to one or more pre-determined threshold values for each of the one or more spirometry measurements Preferably, wherein the processing means and/or external computing device 306 and/or cloud computing device 304 is configured to continuously monitor the user's inhalation characteristics over a period of time and/or a predetermined number of uses of the inhaler and alter the user score(s) based on one or more changes in the user's inhalation characteristics and/or spirometry measurements over the period of time and/or number of inhaler uses and/or number of times spirometry measurements have been acquired. Ideally, the processing means is configured to continuously monitor the user's inhalation characteristics over a period of time and/or number of inhaler uses and alter the one or more pre-determined threshold values for the inhalation characteristics based on one or more changes in the user's inhalation characteristics over the period of time and/or the number of inhaler uses. The period of time is typically a predetermined amount of time, e.g. a week or a month etc. The pre-determined threshold values for the one or more inhalation characteristics may be altered based on one or more user attributes such as but not limited to age, medical condition(s), gender or any other suitable user attribute which may affect the user's inhalation capabilities.

As mentioned previously, the score for each of the inhalation characteristics is typically provided to the user at least via the feedback means 13, with the score for each of the inhalation characteristics being incorporated within the feedback information. Advantageously, this provides the user with a score for each of the inhalation characteristics in real time, displayed on the computing device 1 via the feedback means 209. Additionally or alternatively, the scores for each of the inhalation characteristics may also be transmitted via wired or wireless transmission means to an external computing device such as the user's personal computing device e.g. a smartphone or tablet 306.

In a preferred embodiment, the scores for each of the inhalation characteristics for the user and/or the user's inhalation characteristics and/or one or more of the spirometry measurements or any other measurements acquired by the one or more sensors 15 will be automatically transmitted by the wireless transmission means, typically Bluetooth ^{®}, included within the inhaler monitoring device 1, 150 to an external computing device and/or directly or indirectly to a cloud based server. The external computing device 306, as mentioned previously, typically comprises the user's personal computing device or the personal computing device of a guardian or carer. The personal computing device of the user is configured to provide a display means by which the user may view and optionally interact with the feedback provided by the inhaler monitoring device 1, 150. To this end the personal computing device may be provided or required to be provided with a software application, an "app", which is configured to receive the data transmitted by the inhaler monitoring device 1 and display this to the user in a predetermined manner. Accordingly, the external computing device and/or server will therefore provide an additional feedback means 13 for the user. Advantageously, the feedback means 13; in particular the external server is configured to display historical data/notifications, reports etc. regarding the user's inhalation characteristics and scores and/or spirometry measurements. Optionally this may take the form of an RPM type counter display of adherence to performance parameters and inhalation technique scores on the App. Additionally a real time LED display of the scores for each of the inhalation characteristics may also be provided on the inhaler monitoring device 1 as mentioned.

Advantageously, the AI algorithm embedded in the inhaler monitoring device 1 uses artificial intelligence (AI) applied to the sensor data to provide a novel function which allows users to visualise the individual steps of their inhalation technique on an app or other interface, as individual and collated scores, to address areas of poor technique. The purpose of this is to incrementally and quantitatively improve inhalation technique.

Dynamic adaptation of sensitivity of threshold parameters by the algorithm for new users provides additional new training functionality to encourage correct inhalation technique at the start of usage

An aspect of the present disclosure provides an inhaler monitoring system 300 (as shown in Figure 9) comprising at least: the inhaler apparatus 301, the inhaler apparatus comprising the inhaler monitoring device 1 and coupled spacer 11, inhaler 7; and an external computing device 306. The external computing device 306 typically comprising the user's personal computing device such as their smartphone or the like. The inhaler monitoring system may further comprise a remote computing device which is configured to receive and/or transmit data to the external computing device 306 and/or the inhaler monitoring device 1 of the inhaler apparatus. The remote computing 302 device typically comprises a central server 304 which typically resides in the cloud, a cloud based server (i.e. cloud device as shown in Figure 9). Once the user has finished their inhalation and the data in relation to this has been transmitted from the inhaler monitoring device 1 to the external computing device 306; the external computing device 306 is typically then configured to automatically transmit this data to the server for further analysis. To this end the server may be configured to apply further Al algorithms to the data received from the external computing device such as but not limited to: Amazon SageMaker, Microsoft Azure ML Services, Google Cloud ML Engine and IBM Watson Studio. Similarly with respect to the inhaler monitoring device 150, once the user has typically performed at least three exhalations, the data acquired by the inhaler monitoring device 150 may be transmitted to the external computing device 306; which may further process the data itself and/or automatically transmit this data to the cloud server 304 for further analysis.

The central or cloud based server 304 will effectively consolidate user inhalation characteristics (shake duration, dispense time, duration between dispense and inhalation, inhalation rate, medication dose, time stamp, location of usage, journal entries) with further supplemental data such as spirometry measurements. The further supplemental data may comprise additional other clinical/ physiological data (medication regime, FeNO, FEV, IgE, age, weight, hospitalisation/ exacerbation history) and environmental information (air quality, pollen index, temperature, humidity, respiratory virus alerts etc). The server may be configured to perform one or more predetermined actions based on the received user inhalation characteristics and/or spirometry measurements and/or the supplemental data for the user. The one or more predetermined actions may comprise a non-adherence action, a risk action and/or a monitoring action. The external computing device may also be pre-programmed to provide the non-adherence action, risk action and/or monitoring action based on the user's inhalation characteristics and/or spirometry measurements without communicating with the server.

Advantageously the user's inhalation characteristics and/or spirometry measurements may also be made available to the user's clinician via the central server, for example the user's doctor may be able to access the user's inhalation characteristics and/or spirometry measurements, typically via a website, using their own computing device 305 or the like, to view the user's inhalation characteristics and/or spirometry measurements and further preferably initiate contact with the user based on this data.

The non-adherence action may comprise where the server instructs the external computing device to provide one or more notifications to the user based on their inhalation characteristics. The notification is preferably user specific as the server, in particular the Al algorithms utilised thereby, are configured to automatically reason across the data provided by the inhaler monitoring device 1 and identify patterns of non-adherence and provide both patient and clinician interventions, as appropriate to risk level. The central server undertakes analysis of the immediate (short term) adherence (inhalation performance and prescription adherence data).

For example, when the user has finished inhaling their dispensed medication using the inhaler apparatus, the external computing device may be configured to display a video highlighting any non-adherence events which could be improved upon to provide optimum user inhalation characteristics. To this end at the end of each inhalation, the user's smartphone or other portable device may be configured to launch a short video on the app highlighting where non-adherence occurred. For example, if the user did not shake or the shake duration was too short, then this non- adherence event is highlighted on the video (as an alert/prompt to the user). Table 1 defines a plurality of non-adherence actions which the server may be configured to instruct the external computing device to perform where cases of non-adherence occur. Additionally or alternatively the external computing device may be pre-programmed to automatically perform one or more of the below non-adherence actions in response to the user inhalation characteristics received from the inhaler monitoring device 1 without the need for contacting the server or in instances where it is not possible to communicate with the server. The table details a plurality of examples of non-adherence events which may occur, the means by which the server and/or external computing device is configured to interact with the user and the audience or recipient of the interaction.

**Table 1: Non- adherence Actions**

| **#** | **Non- adherence event** | **Intervention medium** | **Audience** |
|---|---|---|---|
| 1 | Medication not taken as prescribed | App notification stating non-adherence issue. | User HCP (Health Care professional) after a predefined number of consecutive missed events |
| 2 | No shake/not shaken for long enough | App notification stating non-adherence. Provide an animation showing correct use shake duration (adherence). | User |
| 3 | Inhalation rate too fast | App notification stating non-adherence. Provide an animation showing correct inhalation rate (adherence). | User |
| 4 | Inhalation rate too slow | App notification stating non-adherence. Provide an animation showing correct inhalation rate (compliance). | User |
| 5 | Chamber not fully emptied | Animation to App showing the non- adherence issue. Animation shows device use, i.e. when the chamber is emptied. | User |
| 6 | Medication change issue | HCP must change registration data to include new medicines | User |
| | | | HCP |
| 7 | Time between actuation and inhalation too long | App animation stating non-adherence. Provide video showing correct procedure between shake & initial inhalation | User |
| 8 | Not fully engaged with mouthpiece/mask | Notification to the App showing the non-adherence issue - engage with the mouthpiece. | User |
| 9 | Data is not complete from the device | App notification to check device is charged. | User, HCP |
| 10 | Weekly summary trend report | Graphical report to App and web. | User |
| | | Automated messages to improve adherence generated from daily data | HCP |
| 11 | Adherence score very low | HCP advice or App media data e.g. video or animation etc. | HCP |
| | | | User |

The risk action which the server and/or external computing device may be configured to provide typically comprise a risk notification indicating the current condition of the user's medical condition based on the user's inhalation characteristics and/or spirometry measurements. The risk notifications may be provided to the user via the app on the external computing device in-use. The risk notification may include user prompts to take medication, and/or specific times which to take the medication. The central server and/or external computing device may be configured to determine one or more trends based on the user's inhalation and/or spirometry measurements and alter the risk notification accordingly over time. For example, the central server may be configured to determine an adherence trend profile over time for each user which aggregates with environmental data and a risk level for exacerbation is determined by comparing with benchmark data for people with the same medical condition and/or the same age, gender etc. as the user. A personalised exacerbation threshold may be defined to facilitate the time and nature of prompts.

Table 2 illustrates a plurality of examples of risk notifications which may be provided to the user including the risk of the user's condition exacerbating, the current trend of the user's condition defines the prediction types, possible outcomes and the target audience for prompts.

**Table 2: Notification Types**

| **Risk notifications** | **Outcome** | **Audience** |
|---|---|---|
| Exacerbation/ symptom worsening | Risk level Low, Medium or High | Daily to user; Risk threshold breach, send to HCP |
| Adherence trends | Positive progress (Good); | Daily to user; Negative |
| Prescription adherence | Decline in progress (Poor); | progress only to HCP by threshold breach |
| Inhalation adherence | Neutral progress (no change) | |

The central or cloud based server and/or the external computing device provides a storage repository and a seamless reporting capability, via graphics/prompts, on adherence and inhalation technique performance. The output data from the server and/or external computing device relates to interventions which are typically presented to the user via the feedback means 13, which may include the external computing device itself wherein the interventions will be presented to the user via the software application provided thereon. The server and/or the external computing device is typically configured to notify the user's health care provider (HCP) when a user's risk reaches the exacerbation threshold - or if risk has remained consistently high over a prolonged period. Advantageously, the information regarding the user provided by the inhaler monitoring device 1 including the inhalation characteristics and optionally the spirometry measurements acquired by the inhaler monitoring device 150 is also available via the server, typically using a web-based reporting interface (I/F) or the like, to a remote party such as the user's clinician. The clinician can access the server, typically via the web-based interface, to view and interact with the information obtained and/or determined for the user. For example, the web-based clinician I/F has the capability to feedback to the server with a *'teacher'* signal to re-enforce any 'correct' predictions by the server, and similar to identify any predictions deemed 'incorrect". This provides a basis for human-in-the-loop feedback to support training of the machine learning/Al algorithms. The external computing device typically comprises a software application which is configured to display a message board for patients/guardians and facilitates registration of new patients. The message board will contain daily updates on health advice pertaining to specific medical conditions such as asthma and trends on good practice and links to educations media. The server is also typically configured to record the purpose and nature of prompts to be issued to the user as well as: appointments with clinicians; trigger risk and/or display the user's adherence and inhalation technique score.

The disclosure also provides a method for monitoring inhaler technique competence, the method comprising:
Receiving one or more inhalation characteristics of a user;
Determining feedback information based on the one or more inhalation characteristics of the user; and
Providing the feedback information to the user.

The method for monitoring inhaler technique competence is a computer implemented method, wherein the received inhalation characteristics of the user typically comprises data indicative of the inhalation characteristics of the user. The step of receiving one or more inhalation characteristics typically comprises received by the processing means of the inhaler monitoring device and/or the external or remote computing devices receiving the inhalation characteristics from the inhaler monitoring device as described previously (recited in claim 1). The step of determining feedback information based on the one or more inhalation characteristics of the user; typically comprises determining by the inhaler monitoring device, in particular the processing means thereof, the feedback information, however this may also be performed by the external or remote computing devices. The method can be performed offline, with each of the method steps being performed locally on the inhaler monitoring device 1. Wherein determining the feedback information based on the one or more inhalation characteristics of the user may comprise calculating a user score based on the user's inhalation characteristics. Ideally, a separate user score is determined in respect of the each of the different inhalation characteristics of the user. The user score is determined based on the user's inhalation characteristics with respect to one or more pre-determined threshold values for the one or more inhalation characteristics. The method may further comprise monitoring the user's inhalation characteristics over a period of time and/or number of inhaler uses and altering the user score(s) based on one or more changes in the user's inhalation characteristics over the period of time and/or number of inhaler uses. To this end the method may further comprise monitoring the user's inhalation characteristics over a period of time and/or number of inhaler uses and altering the one or more pre-determined threshold values for the inhalation characteristics based on one or more changes in the user's inhalation characteristics over the period of time and/or number of inhaler uses. The pre-determined threshold values for the one or more inhalation characteristics may vary based on one or more user attributes such as age, medical condition(s), gender or any other suitable user attribute.

Further as described previously the existing software provided on the computing device, including for example aflo^{™} firmware and cloud software, may be adjusted to accommodate this new spirometry functionality. A spirometry reading would be prompted when the decision making cloud based algorithm, implemented by the processing device of the spirometry functionality 150 and/or the computing device and/or the external software, detects a deterioration in the patient's inhaler technique, adherence to prescribed inhaled medication and/ or symptom control (as already measured on the aflo^{™} platform), or on a regular basis or at any time of the patient/respiratory consultant choosing. Spirometry data, and spirometry measurements determined therefrom, recorded over time by the aflo^{™} device would be processed by the cloud algorithm along with longitudinal inhalation and environmental data. The inclusion of this data would drive patient alerts and additionally maintain an electronic patient diary that relates their condition to one of more of the five steps of inhalation and/or changes in the environmental data. Specifically, the central server would be configured to consolidate the feedback information provided by the aflo^{™} device (user inhalation characteristics data with one or more of the spirometry measurements such as Peak Expiratory Flow, Forced Vital Capacity or Forced Expiratory Volume score with all supplemental data to determine an advisory action based on the consolidated data. Real time values for the spirometry measurements would be available to patients by means of the computing device of the user (i.e. external computing device 306), in particular via the software application pre-installed therein such as the aflo^{™} app and to clinicians via a similar pre-installed software application on their own computing device or via a web browser portal such as the aflo^{™} clinical portal.

Referring now to Figures 14 and 15 there is shown a flow diagram illustrating an inhalation algorithm embodying a further aspect of the present disclosure and a diagram illustrating five steps of optimal inhalation technique and the associated time/ duration/ speed or volume measurement for each of the steps which aim to automate inhalation technique steps for pressurised metered dose inhalers, using the algorithm reflecting the relative impact of steps of inhalation on lung deposition. A widely used method for delivery of medication to treat asthma, Chronic Obstructive Pulmonary Disease (COPD) and other respiratory diseases is by pressurised Metered Dose Inhaler (pMDI/ MDI). This method of delivery requires a co-ordinated series of actions by the patient to ensure that the medication within the aerosol from the MDI is deposited deep in the airways to permit optimal symptom control (lung deposition).

The most commonly used method of improving the efficacy of medication delivery from MDI is the use of a Valved-Holding-Chamber (VHC) or spacer device in conjunction with the MDI such as is detailed earlier in the application. The process of using a conventional MDI/spacer device combination requires instruction from a Health Care Professional (HCP) to users on performing a series of actions that are challenging both technically and, in their co-ordination, and sequencing. Evidence shows that poor user technique is a global problem (86% inhalers users make at least one error, Usmani, et al) which is thought to be caused by difficulty in mastering the correct inhalation technique, remembering the series of steps, and poor user engagement and insight into the benefits of optimal inhalation technique. MDI device combinations with a data capture device comprising integrated sensors that can capture data indicative of medication adherence have recently become available, e.g. smart inhaler, Propellor Health, CoHero, CapMedic, PuffClicker. These inventions attach directly to the MDI and send user information to a connected app and/or clinician accessible portal/ dashboard. However, they do not attach to a spacer so they cannot enable the full monitoring and subsequent display to users of all five essential inhalation steps, unlike the embodiments of the present disclosure such as the inhaler monitoring device shown in Figures 1 to 5, to allow optimal medication deposition to control respiratory symptoms. Uniquely, the inhaler monitoring device 1 (referred to in practice as aflo^{™}) platform offers this functionality as described previously. Data analysis of sensor information to provide informed user and HCP feedback, and the setting of inhalation performance adherence metrics, is needed to ensure a data driven, hyper-personalised approach.

The present embodiment relates to applying a weighted decision methodology algorithm to the one or more inhalation characteristics determined based on data acquired by the inhaler monitoring device 1. The weighted decision methodology algorithm is typically implemented by the processing means of the inhaler monitoring device 1, 303 and/or the external computing device 306 including the user's personal computing device and/or the cloud device 304 based on data acquired by the inhaler monitoring device typically including recent and historical data. The one or more inhalation characteristics including but not limited to one or more of the five key steps of inhalation technique when using an MDI with a spacer device. The purpose is to provide quantified real-time feedback to patients, focusing on the measurable impact of each inhalation step, to correct pMDI technique and enable the optimisation of medication deposition in the lungs, maximising symptom control.

The five critical inhalation steps are:
1. inhaler shake duration (S1);
2. time interval between shake and the dispense of the medication (S2);
3. interval between dispense of the medication into the spacer chamber and inhalation commencing (S3);
4. inspiratory flow rate (S4);
5. volume of medication inhaled (S5);

One or more further steps (S6 to Sn which may include full exhalation and breath hold post inhalation) may influence lung deposition and may or may not be included in the audio visual guidance provided on an external computing device. Existing evidence demonstrates that core inhaler technique errors relating to: 1. the duration of medication agitation, 2. the time interval between the end of agitation and the dispense (so called 'shake to fire interval), 3. the time interval between dispense occurring and inhalation commencing, 4. The speed of inhalation; and 5. the volume of medication inhaled, i.e. the five critical inhalation steps as defined previously all have an impact on the amount of medication that is deposited in the lungs. The presence of these individual or combined errors has an negative impact on respiratory symptom control.

Correct use of an inhaler (pMDI) requires the correct achievement of the steps listed above to achieve effective drug delivery. These steps are generally the same between different pMDIs due to the similar device designs and operating principles. For suspension formulations in particular, shaking the pMDI is an essential step to ensure that the aerosol released from the device contains a uniform drug dose. Research indicates that solution based pMDIs remain stable over a range of shake to fire intervals. As most patients and doctors would not know whether the drug is in solution or suspension, it has become a universal instruction to shake any pMDI before use. Research confirms that guidance should be given to users/caregivers on the timing of firing after shaking their device, and in particular to paediatric patients and those who require support to manage their condition.

The timing between shaking the pMDI and actuating a dose (shake - fire interval) is rarely specified in the patient instruction leaflet of the prescribed pMDI, and is an important area of research, as it has already been shown that suspension formulations within an HFA pMDI can cream or sediment soon after the device has been shaken due to density differences between the drug and the propellant, and this has also been observed in a clinical setting.

Data available on the subject of creaming and sedimentation of pMDI drug formulations currently in use are limited, especially with respect to the acceptable length of time between shaking the device and actuating a shot. Simple mistakes in pMDI use, such as dropping the pMDI, being distracted and difficulties in attaching the pMDI to a spacer, can increase the time between shaking the device and actuating a dose.

Spacers constitute a constrained volume chamber into which a patient actuates the pMDI and from which the patient inhales. While spacer usage helps to avoid errors of coordination between actuation and the start of inhalation, they introduce new patient errors such as the possibility of delay between pMDI actuation and inhalation from spacer. It has been reported that a continual reduction in drug delivery coincided with an increasing delay time between actuation and the start of inhalation: specifically a 20 second delay time reduced drug delivery by two-thirds. To avoid excessive settling of the medication to the bottom of the spacer it is now widely accepted that the user should engage in the inhalation process within 5 seconds of the time of dispense

Data available on inspiratory flow rates for pMDls are limited. However, several studies have been carried out with no general agreement of optimal flow rate as this depends on the combination of pMDI and spacer in use. Reading across the literature it appears that there is a consensus on flow rate which suggests a minimal rate of 15L/min with a maximum rate of 120L/min. Since most devices on the market are generic and product specific data are available for only a few drugs, it is currently not possible to determine whether for some systems a higher minimum required inspiratory flow rate should be recommended. However, to achieve optimum deposition a flow rate between 30L/min to 60L/min is recommended clinical practice.

Small volume spacers, which tend to be less than 100 mL, are tube-like extensions of the mouthpiece of the pMDI but without any unidirectional valve. They are much less cumbersome than the larger spacers but require a greater need for additional coordination between actuation of the pMDI and commencement of inhalation. Medium and large volume spacers fall in the ranges of 100-350 mL and >700 mL respectively and usually incorporate a unidirectional valve at their mouthpiece end, allowing inhalation from the spacer. These devices are called VHCs and allow more leeway in the time available to the patient to commence inhalation after activating the pMDI: VHC/spacers also allow for tidal breathing. When breathing in from a spacer/VHC, a single slow and deep inhalation followed by a breath hold is optimal where a minimum flow rate of 15 L/min is acceptable while between 30 and 60L/min is optimal. Therefore, patient inhalation duration can be linked to their inhalation flow by equating the product of inhalation flow rate and inhalation duration to the volume of the spacer in use.

To provide quantified real-time feedback to patients, which relates the impact of each inhalation step to drug deposition, the present method is proposed that can weight each step according to its relative level of impact on measurable drug deposition.

Referring now in particular to Figure 15, there is shown generally indicated by the reference numeral 500, n steps of inhalation S1 to Sn, wherein each step is weighted independently with weights W1 to Wn. Note that each weight directly reflects the impact of the associated inhalation step on drug deposition. Furthermore, an overall score S is assigned to the inhalation process where the contribution of each weighted step is reflected in S: note that S is indicative of the level of lung deposition. Each weight W1 to Wn will be in the range 0 to 1 where 1 reflects maximum impact and each step S1 to Sn is assigned values of between 0 and 1 where 1 denotes an optimal score in that step (See Fig. 1). We can now write that these quantities are related by the algorithm according to; $S = \frac{{\sum }_{1}^{n} SnWn}{{\sum }_{1}^{n} Wn}$

S will be in a range 0 to 1 (or 0 to 100%) where the contribution of Sn to S is reflected by the impact weighting Wn. For example, in the embodiment described and shown for example in Figures 14 and 15, there are five steps S1 to S5 where each step is assigned a score comprising either an optimal, sub-optimal and critical score depending on how well the user performs the step. In another scenario these steps may be expanded to include other physiological activities pertaining to lung deposition, being steps S1 to Sn. In the example scenario below, the scores will be mapped to alphanumeric numbers in the range 1 to 0 respectively. Additionally, as new real world data emerges on the relative impact of each step to drug deposition then this may be reflected in adjusted relative weightings in the above algorithm.

Accordingly the method implemented by the present embodiment, in particular the method of optimising inhaler inhalation technique comprises: acquiring, inhalation data using an inhaler monitoring apparatus 1; determining, typically by the inhaler monitoring apparatus 1, one or more inhalation characteristics based on the inhalation data; weighting each of the one or more inhalation characteristics, preferably wherein each inhalation characteristics is weighted individually based on the pre-determined impact of the associated inhalation characteristic on drug deposition; and determining an inhalation technique score based on the combination of each of the one or more inhalation characteristics and their respective weighting. Ideally the method further comprises providing the determined inhalation technique score to the user. Preferably, wherein the one or more inhalation characteristics include at least one of, preferably all, inhaler shake duration 501 (S1); Time interval between shake and the dispense of the medication 502 (S2); 3. Interval between dispense of the medication into the spacer chamber and inhalation commencing 503 (S3); 4. Inspiratory flow rate 504 (S4); and 5. Volume of medication inhaled 505 (S5). Ideally, wherein weighting each of the one or more inhalation characteristics comprises determining for each of the: shake duration 501, shake to dispense interval 502 and dispense to inhale 503, the respective critical, sub optimal and optimal time periods for the effectiveness of each of the inhalation characteristic.

For example, as shown in Figure 15 it is critical that the shake duration 501 lasts for greater than 0 seconds, sub-optimal if it lasts from greater than 0 to 5s and optimal if the shake duration occurs for between 5s to 5.5s. Similarly it is optimal when the shake to dispense interval 502 lasts from 1s to 5s, sub optimal from 5s to 10s and critical when it lasts for greater than 10s. Further it is optimal if the time for dispense to inhale 503 has a duration from 0s to 4s, sub optimal if the duration is from 4s to 5s and critical if the duration is greater than 5s. Further, ideally, wherein weighting each of the one or more inhalation characteristics comprises determining for the inspiratory flow rate 504, the critical, sub optimal and optimal flow rate. For example it is optimal if the inspiratory flow rate is a value within the range from 20L/min to 60L/min, sub optimal if the inspiratory flow rate is from greater than 0L/min to 20L/min or greater than 60L/min and critical if the inspiratory flow rate is 0L/min. Further, preferably, wherein weighting each of the one or more inhalation characteristics comprises determining for the volume inhaled 505, the critical, sub optimal and optimal volume. For example the optimal volume inhaled is within the range from 150mL to 300mL, sub optimal is from a value greater than 0ml to 149mL and critical if the volume is 0mL.

It should be understood that the above values are provided for the purposes of an example are not intended to be limiting and the values detailed above may vary in practice for example depending on the particular inhaler and/or medication being dispensed.

Referring now to Figure 14, there is shown a flow diagram illustrating the steps for the method of optimising inhaler inhalation technique generally indicated by the reference numeral 400. The method as mentioned previously, is typically implemented by the processing means 303 of the inhaler monitoring device 1 and/or the external computing device 306 including the user's personal computing device and/or the cloud device 304 based on data acquired by the inhaler monitoring device 1, in particular the sensors thereof. The method may comprise more or less steps than S1 to S5 as is defined, with each step relating to a particular inhalation characteristic such that the number of steps may comprise St number of steps.

The respective processing means will first read all sensor values and timestamps user engagement at each step, in a specified chronological sequence which reflects the required correct user behaviour to achieve optimal lung deposition with inhaled medication.

The monitored timestamps are as follows:
Tss = time of shake (agitation of medication in the inhaler canister) start;
Tsf = time of shake stop;
Td = time of dispense (pressing the top of the inhaler canister to release medication);
Tis = time of inhalation start.

Once Tss, Tsf, Td and Tis have been read in 401 the various steps of inhalation can be calculated. There are five steps of inhalation, as mentioned previously in relation to Figure 15 and each step can be calculated as follows:
First S1, S2 and S3 are calculated 402,
**Step 1 Shake Duration 501** (S1) is the time difference between the time of shake start and shake stop,
Therefore S1= Tsf - Tss
**Step 2 Shake to Dispense Interval 502** (S2) is the time difference between shake stop and time of dispense,
Therefore S2 = Tsf-Td
**Step 3 Dispense to Inhale 503** (S3) is the time difference between the dispense action and the start of inhalation,
Therefore S3 = Td - Tis

The sensor values are read in and S4 and S5 are calculated,
**Step 4 Inspiratory Flow rate 504** (S4) measures the speed at which the medication is inhaled and calculates the average inhalation rate,
**Step 5 Volume Inhaled 505** (S5) measures the total volume of medication inhaled,

Once S1 to S5 are calculated a value is assigned to each of the steps in the range of 0 to 1 404. Further a weighting is then assigned to each step 405 (W1 to W5), wherein each weight directly reflects the impact of the associated inhalation step on drug deposition. An **overall score (S)** is then assigned to the inhalation process 406, where the contribution of each weighted step is reflected in S, wherein S is indicative of the level of lung deposition.

S is calculated as the sum of all weighted steps (eg, W1.S1+W2.S2+W3.S3+W4.S4 +W5.S5) and the maximum sum of all weights must add to unity (eg, W1+W2+W3+W4+W5=1) and therefore each step is assigned a value between 0 and 1: 1 is where a step is performed perfectly correctly.

Subsequently, each of the calculated values (S1 to St) and the overall score are provided to the user 407, typically via the pre-installed app or web interface, such that the user can work to improve each of the calculated values and/or the overall score to optimise their inhaler inhalation technique. The calculated values and/or the overall score may further be enhanced with supplemental data including for example spirometry measurements and/or environmental data.

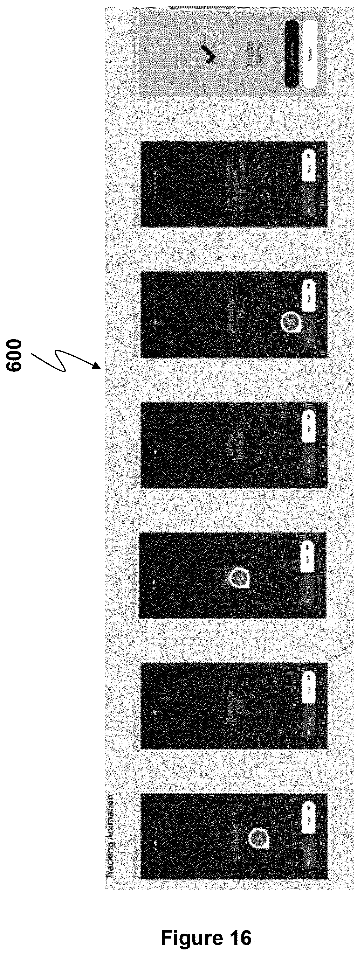

Referring now to Figure 16 there is shown, generally indicated by the reference numeral 600, a sequence of images of a graphical user interface (GUI) which embodies a further aspect of the present disclosure. Accordingly this aspect provides a method of guiding optimal inhalation technique, in particular across one or more of the five steps of inhalation S1 to S5 such as are shown in Figure 15 and which are calculated in Figure 14. The method comprises providing prompts to the user based on the thresholds for the five steps of inhalation S1 to S5 shown in Figure 15. In Figure 16 this takes the form of the GUI which may be configured to display a visual prompt to the user according to the thresholds determined for the five steps of inhalation however it may additionally or alternatively take the form of other visual or audio feedback which may be provided to the user. For example the visual or audible feedback may be provided to the user via the external computing device 306, typically using the display and/or speakers thereof. The external computing device 306, in particular the GUI shown thereon, is configured to automate the presentation of audio-visual cues presented on the external computing device 306 in a manner which demonstrates to users the correct sequence and/or timing of events for optimised inhalation of medication via an MDI using the inhaler monitoring device 1.

In use, the inhaler monitoring device 1 will preferably be communicatively coupled via wireless transmission means, in particular short wave low power transmission means such as Bluetooth^{®}, to the external computing device 306, preferably comprising the user's own smartphone or tablet, having a pre-installed software application, as described previously, provided thereon which is configured to display the GUI as shown in Figure 16. The GUI is configured to display prompts for optimal inhaler technique based on data provided by the inhaler monitoring device 1 in particular the one or more sensors thereof and/or data or measurements determined based on data acquired by the inhaler monitoring device 1. For example the inhaler monitoring device 1 preferably includes a movement sensor typically comprising an accelerometer, which is configured to measure when the inhaler monitoring device is being shaken or otherwise agitated (S1), this sensor can accordingly measure how long the inhaler monitoring device 1 is shaken prior to dispensation and transmit this data in real time to the external computing device 306 such that the GUI displays feedback indicating to the user whether to shake the user monitoring device 1 longer or whether to move onto the next step of the five steps of inhalation. For example the GUI may be configured to initially display a "shake" prompt until 5 seconds of shaking of the inhaler monitoring device 1 has been detected using the accelerometer of the inhaler monitoring device 1 upon which time the GUI will display a prompt according to the next step. Subsequently the pressure sensor may be configured to provide data regarding the subsequent inhalation steps (S2) to (S5) with the GUI altering the displayed visual prompt accordingly. For example when each of the following are detected by the pressure sensor: when dispensation of the medication from inhaler occurs (S2); when inhalation from dispensation (S3) starts; how fast the inspiratory flow rate (S4) is; and when the full volume of medication has been inhaled (S5). Advantageously the timing for each of the displayed prompts on the GUI will correspond to the optimal values as shown for example in Figure 15 such that the user is guided efficiently and quickly through the five steps of inhalation (S1 to S5) in the most optimum manner possible.

It will be understood that what has been described herein is an exemplary inhaler monitoring device and inhaler monitoring system. While the present teaching has been described with reference to exemplary arrangements it will be understood that it is not intended to limit the teaching to such arrangements as modifications can be made without departing from the scope of the present teaching.

It will be understood that while exemplary features of a distributed network system in accordance with the present teaching have been described that such an arrangement is not to be construed as limiting the disclosure to such features. The method of the present teaching may be implemented in software, firmware, hardware, or a combination thereof. In one mode, the method is implemented in software, as an executable program, and is executed by one or more special or general purpose digital computer(s), such as a personal computer (PC; IBM-compatible, Apple-compatible, or otherwise), personal digital assistant, workstation, minicomputer, or mainframe computer. The steps of the method may be implemented by a server or computer in which the software modules reside or partially reside. Generally, in terms of hardware architecture, such a computer will include, as will be well understood by the person skilled in the art, a processor, memory, and one or more input and/or output (I/O) devices (or peripherals) that are communicatively coupled via a local interface. The local interface can be, for example, but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the other computer components. The processor(s) may be programmed to perform the functions of the first, second, third and fourth modules as described above. The processor(s) is a hardware device for executing software, particularly software stored in memory. Processor(s) can be any custom made or commercially available processor, a central processing unit (CPU), an auxiliary processor among several processors associated with a computer, a semiconductor based microprocessor (in the form of a microchip or chip set), a microprocessor, or generally any device for executing software instructions.

Memory is associated with processor(s) and can include any one or a combination of volatile memory elements (e.g., random access memory (RAM, such as DRAM, SRAM, SDRAM, etc.)) and non-volatile memory elements (e.g., ROM, hard drive, tape, CDROM, etc.). Moreover, memory may incorporate electronic, magnetic, optical, and/or other types of storage media. Memory can have a distributed architecture where various components are situated remote from one another, but are still accessed by processor(s).

The software in memory may include one or more separate programs. The separate programs comprise ordered listings of executable instructions for implementing logical functions in order to implement the functions of the modules. In the example of heretofore described, the software in memory includes the one or more components of the method and is executable on a suitable operating system (O/S).

The present teaching may include components provided as a source program, executable program (object code), script, or any other entity comprising a set of instructions to be performed. When a source program, the program needs to be translated via a compiler, assembler, interpreter, or the like, which may or may not be included within the memory, so as to operate properly in connection with the O/S.

Furthermore, a methodology implemented according to the teaching may be expressed as (a) an object oriented programming language, which has classes of data and methods, or (b) a procedural programming language, which has routines, subroutines, and/or functions, for example but not limited to, C, C++, Pascal, Basic, Fortran, Cobol, Perl, Java, Json and Ada.

When the method is implemented in software, it should be noted that such software can be stored on any computer readable medium for use by or in connection with any computer related system or method. In the context of this teaching, a computer readable medium is an electronic, magnetic, optical, or other physical device or means that can contain or store a computer program for use by or in connection with a computer related system or method. Such an arrangement can be embodied in any computer-readable medium for use by or in connection with an instruction execution system, apparatus, or device, such as a computer-based system, processor-containing system, or other system that can fetch process the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be for example, but not limited to, an electronic, magnetic, optical, electromagnetic, infrared, or semiconductor system, apparatus, device, or propagation medium. Any process descriptions or blocks in the Figures should be understood as representing modules, segments, or portions of code which include one or more executable instructions for implementing specific logical functions or steps in the process, as would be understood by those having ordinary skill in the art.

It should be emphasized that the above-described embodiments of the present teaching, particularly, any "preferred" embodiments, are possible examples of implementations, merely set forth for a clear understanding of the principles. Many variations and modifications may be made to the above-described embodiment(s) without substantially departing from the principles of the present teaching. All such modifications are intended to be included herein within the scope of this disclosure.

The invention is not limited to the embodiment(s) described herein but can be amended or modified without departing from the scope of the present invention which is defined by the claims which follow.

## Claims

1. An inhaler monitoring system for monitoring inhaler technique competence, guiding optimal inhalation technique of a user and improving inhaler technique comprising
an inhaler (9) configured to dispense medication;
a spacer (11);
an inhaler monitoring device (1), an external computing device, and a remote computing device;
wherein the inhaler monitoring device comprises:
a body (2) comprising an inlet (3) and an outlet (5) which are in fluid communication with respect to one another to define a channel therebetween;
wherein the inlet is for coupling to the inhaler (9) for dispensing a medication and the outlet is for coupling to the spacer (11) through which a user can inhale the dispensed medication from the inhaler;
one or more sensors (15) which are configured to detect and measure inhalation characteristics when the user inhales the dispensed medication from the inhaler through the inhaler monitoring device via the spacer in use; and
processing means which is configured to determine feedback information based on the inhalation characteristics of the user:
**characterised in that** the inhaler monitoring device comprises wireless transmission means configured to transmit data regarding the inhalation characteristics to the external computing device and/or the remote computing device, wherein one or more of the processing means, external computing device and remote computing device is configured to apply an Al algorithm to the inhalation characteristics data to determine the user specific feedback, wherein the Al algorithm is trained with the user's inhalation characteristics such that the user specific feedback provided to the user dynamically adapts over time, and to provide the feedback information to the user to guide optimal inhalation technique of the user and improve user inhaler technique in real time; and
wherein the inhalation
characteristics comprise:
airflow characteristics;
threshold and duration and intensity of shake of the inhalation monitoring device, prior to, the dispensation of medication from the inhaler;
the time between end of shake and dispense of medication;
the time between dispensation of medication and start of inhalation;
inhalation rate;
volume of medication inhaled
and
medication dosage information.

2. The inhaler monitoring system of claim 1, further comprising feedback means which is configured to provide visual, audible and/or haptic feedback to the user based on the feedback information and/or one or more of the inhalation characteristics, preferably wherein the feedback means is configured to provide the feedback to the user in real time, and preferably wherein the feedback means comprises a plurality of LEDs which are located on the body of the device, which are configured to illuminate in a predetermined sequence based on the one or more inhalation characteristics of the user.

3. The inhaler monitoring system of any preceding claim, wherein the one or more sensors comprise: at least one air pressure sensor; at least one movement sensor and/or at least one environmental sensor.

4. The inhaler monitoring system of any preceding claim, wherein the feedback information comprises at the least the inhalation characteristics of the user.

5. The inhaler monitoring system of any preceding claim, wherein the feedback information comprises a user score which is determined based on the user's inhalation characteristics, preferably a separate user score is determined in respect of the each of the different inhalation characteristics of the user, wherein the user score is determined based on the user's inhalation characteristics with respect to one or more pre-determined thresholds for the one or more inhalation characteristics.

6. The inhaler monitoring system of claim 5, wherein the processing means is configured to continuously monitor the user's inhalation characteristics over a period of time and alter the user score(s) based on one or more changes in the user's inhalation characteristics over the period of time and/or alter the one or more pre-determined thresholds for the inhalation characteristics based on one or more changes in the user's inhalation characteristics over the period of time.

7. The inhaler monitoring system of claim 5 or 6 wherein the pre-determined thresholds for the one or more inhalation characteristics vary based on one or more user attributes such as age, medical condition(s), gender or any other suitable user attribute.

8. The inhaler monitoring system of any preceding claim, wherein the inhaler monitoring device comprises spirometry functionality to acquire and/or determine spirometry measurements of a user;
wherein the inlet is for coupling to a mouthpiece (8) into which a user can exhale; and
wherein the sensors are configured to acquire spirometry data.

9. The inhaler monitoring system of claim 8 wherein the spirometry measurements comprise peak expiratory flow, forced vital capacity, and/or expiratory volume.

10. The inhaler monitoring system according to any preceding claim
wherein the feedback information comprises media data which is provided to the user by the external computing device, and/or wherein the feedback information comprises a user score, ideally wherein a separate user score is determined for each of the one or more inhalation characteristics of the user, preferably, wherein the user score for each of the inhalation characteristics is dynamically weighted based on one or more of the user's inhalation characteristics.

11. The inhaler monitoring system of claim 10, further comprising a central server which is communicatively coupled to the computing device and/or inhaler monitoring device, wherein the central server is preferably configured to consolidate the feedback information provided by the inhaler monitoring device and/or the user inhalation characteristics with supplemental data to determine an advisory action based on the consolidated user inhalation characteristics and supplemental data, wherein the supplemental data preferably comprises further clinical or physiological data regarding the user, further data regarding the medication being received by the user and/or further environmental information regarding the location where the user made use of the inhaler apparatus and/or third party user data and wherein the advisory action preferably comprises a non-adherence action and/or a risk action, preferably wherein a non-adherence action comprises wherein the central server is configured to communicate with the computing device to notify the user of one or more actions to take to improve their inhalation characteristics, optionally wherein a risk action comprises wherein the central server is configured to communicate with the computing device to notify the user of their risk of their medical condition deteriorating or improving based on their inhalation characteristics.

12. The inhaler monitoring system of claim 10 or 11, wherein the Al algorithm is trained with the user's inhalation characteristics over a period of time and/or supplemental data received from the central server such that the user specific feedback provided to the user dynamically adapts over time; and /or wherein the central server is configured to apply an Al algorithm to the user inhalation characteristics or the user inhalation characteristics and supplemental data, when the central server determines the advisory action.

13. A method for monitoring inhaler technique competence and guiding optimal inhalation technique of a user using an inhaler monitoring system according to any preceding claim, the method comprising:
receiving one or more inhalation characteristics of a user;
determining feedback information based on the one or more inhalation characteristics of the user; and
providing the feedback information to the user;
wherein the feedback information comprises a user score which is determined based on the user's inhalation characteristics;
wherein the wireless transmission means wirelessly transmits data regarding the inhalation characteristics to an external computing device and/or a remote computing device, and wherein one or more of the processing means, external computing device and remote computing device applies an Al algorithm to the inhalation characteristics to to determine the user specific feedback, wherein the Al algorithm is trained with the user's inhalation characteristics such that the user specific feedback provided to the user dynamically adapts over time, and provides the feedback information to the user to guide optimal inhalation technique of the user and improve user inhaler technique in real time;
wherein the inhalation characteristics comprise:
airflow characteristics;
threshold and duration and intensity of shake of the inhalation monitoring device, prior to the dispensation of medication from the inhaler;
the time between end of shake and dispense of medication;
the time between dispensation of medication and start of inhalation;
inhalation rate;
volume of medication inhaled
and
medication dosage information.

14. The method according to claim 13 which comprises receiving one or more spirometry measurements of the user, wherein the spirometry measurements comprise peak expiratory flow, forced vital capacity and/or forced expiratory volume.

## Patentansprüche

1. Inhalator-Überwachungssystem für das Überwachen einer Inhalatortechnik-Kompetenz, für das Anleiten des Benutzers hinsichtlich einer optimalen Inhalationstechnik und für das Verbessern der Inhalatortechnik, umfassend:
einen Inhalator (9), der so konfiguriert ist, dass er ein Medikament abgibt;
ein Abstandsstück (11);
eine Inhalatorüberwachungsvorrichtung (1), ein externes Computergerät und ein Remote-Computergerät;
wobei die Inhalatorüberwachungsvorrichtung umfasst:
einen Körper (2), der einen Einlass (3) und einen Auslass (5) umfasst, die miteinander in Fluidverbindung stehen und dazwischen einen Kanal definieren;
wobei der Einlass zum Koppeln mit dem Inhalator (9) zum Abgeben eines Medikaments und der Auslass zum Koppeln mit dem Abstandsstück (11) vorgesehen ist, durch den ein Benutzer das von dem Inhalator abgegebene Medikament inhalieren kann;
einen oder mehrere Sensoren (15), die so konfiguriert sind, dass sie Inhalationsparameter erfassen und messen, wenn der Benutzer das von dem Inhalator abgegebene Medikament während der Verwendung des Abstandsstücks durch die Inhalatorüberwachungsvorrichtung inhaliert; und
eine Verarbeitungseinrichtung, die so konfiguriert ist, dass sie Feedback-Informationen basierend auf den Inhalationseigenschaften des Benutzers bestimmt;
**dadurch gekennzeichnet, dass** die Inhalatorüberwachungsvorrichtung drahtlose Übertragungsmittel umfasst, die so konfiguriert sind, dass sie Daten bezüglich der Inhalationseigenschadften an das externe Computergerät und/oder das Remote-Computergerät übertragen, wobei eine oder mehrere der Verarbeitungseinrichtungen, des externen Computergeräts und des Remote-Computergeräts so konfiguriert ist bzw. sind, dass sie einen KI-Algorithmus auf die Daten der Inhalationseigenschaften anwenden, um ein benutzerspezifisches Feedback zu bestimmen, wobei der KI-Algorithmus mit den Inhalationseigenschaften des Benutzers trainiert wird, sodass sich das dem Benutzer bereitgestellte benutzerspezifische Feedback im Laufe der Zeit dynamisch anpasst, und die Feedback-Informationen dem Benutzer bereitstellen, um den Benutzer in Echtzeit bei der Durchführung einer optimalen Inhalationstechnik anzuleiten und die Inhalatortechnik des Benutzers zu verbessern; und
wobei die Inhalationseigenschaften Folgendes umfassen:
Strömungseigenschaften;
Schwellenwert, Dauer und Intensität des Schüttelns der Inhalatorüberwachungsvorrichtung vor der Abgabe des Medikaments aus dem Inhalator;
Zeit zwischen dem Ende des Schüttelns und der Abgabe des Medikaments;
Zeit zwischen der Abgabe des Medikaments und dem Beginn der Inhalation;
Inhalationsrate;
Volumen des inhalierten Medikaments und
Dosierungsinformationen zu dem Medikament.

2. Inhalatorüberwachungssystem nach Anspruch 1, ferner umfassend Feedback-Mittel, die so konfiguriert sind, dass sie dem Benutzer basierend auf den Feedback-Informationen und/oder einer oder mehrerer der Inhalationseigenschaften visuelles, akustisches und/oder haptisches Feedback bereitstellen, wobei die Feedback-Mittel vorzugsweise so konfiguriert sind, dass sie dem Benutzer das Feedback in Echtzeit bereitstellen, und wobei die Feedback-Mittel vorzugsweise eine Vielzahl von LEDs umfassen, die am Körper der Vorrichtung angeordnet und so konfiguriert sind, dass sie basierend auf der einen oder den mehreren Inhalationseigenschaften des Benutzers in einer vorbestimmten Sequenz aufleuchten.

3. Inhalatorüberwachungssystem nach einem der vorhergehenden Ansprüche, wobei der eine oder die mehreren Sensoren Folgendes umfassen: mindestens einen Luftdrucksensor; mindestens einen Bewegungssensor und/oder mindestens einen Umgebungssensor.

4. Inhalatorüberwachungssystem nach einem der vorhergehenden Ansprüche, wobei die Feedback-Informationen mindestens die Inhalationseigenschaften des Benutzers umfassen.

5. Inhalatorüberwachungssystem nach einem der vorhergehenden Ansprüche, wobei die Feedback-Informationen eine Benutzerbewertung umfassen, die basierend auf den Inhalationseigenschaften des Benutzers bestimmt wird, wobei vorzugsweise eine separate Benutzerbewertung für jede der verschiedenen Inhalationseigenschaften des Benutzers bestimmt wird, wobei die Benutzerbewertung basierend auf den Inhalationseigenschaften des Benutzers in Bezug auf einen oder mehrere vorbestimmte Schwellenwerte für den einen oder die mehreren Inhalationseigenschaften bestimmt wird.

6. Inhalatorüberwachungssystem nach Anspruch 5, wobei die Verarbeitungseinrichtung so konfiguriert ist, dass sie die Inhalationseigenschaften des Benutzers über einen Zeitraum kontinuierlich überwacht und die Benutzerbewertung(en) basierend auf einer oder mehreren Änderungen der Inhalationseigenschaften des Benutzers über den Zeitraum ändert und/oder die einen oder die mehreren vorbestimmten Schwellenwerte für die Inhalationseigenschaften basierend auf einer oder mehreren Änderungen der Inhalationseigenschaften des Benutzers über den Zeitraum ändert.

7. Inhalatorüberwachungssystem nach Anspruch 5 oder 6, wobei die vorbestimmten Schwellenwerte für die eine oder die mehreren Inhalationseigenschaften basierend auf einem oder mehreren Benutzermerkmalen, wie beispielsweise Alter, medizinische(r) Zustand/Zustände, Geschlecht oder ein beliebiges anderes geeignetes Benutzermerkmal, variieren.

8. Inhalatorüberwachungssystem nach einem der vorhergehenden Ansprüche, wobei die Inhalatorüberwachungsvorrichtung eine Spirometrie-Funktion zum Erfassen und/oder Bestimmen von Spirometrie-Messungen eines Benutzers umfasst;
wobei der Einlass zum Koppeln mit einem Mundstück (8) vorgesehen ist, in das ein Benutzer ausatmen kann; und
wobei die Sensoren so konfiguriert sind, dass sie Spirometriedaten erfassen.

9. Inhalatorüberwachungssystem nach Anspruch 8, wobei die Spirometrie-Messungen den exspiratorischen Spitzenfluss, die forcierte Vitalkapazität und/oder das exspiratorische Volumen umfassen.

10. Inhalatorüberwachungssystem nach einem der vorhergehenden Ansprüche,
wobei die Feedback-Informationen Mediendaten umfassen, die dem Benutzer durch das externe Computergerät bereitgestellt werden, und/oder wobei die Feedback-Informationen eine Benutzerbewertung umfassen, wobei vorzugsweise für jede der einen oder mehreren Inhalationseigenschaften des Benutzers eine separate Benutzerbewertung bestimmt wird, wobei vorzugsweise die Benutzerbewertung für jede der Inhalationseigenschaften dynamisch basierend auf einer oder mehreren der Inhalationseigenschaften des Benutzers gewichtet wird.

11. Inhalatorüberwachungssystem nach Anspruch 10,
ferner umfassend einen zentralen Server, der kommunikativ mit dem Computergerät und/oder der Inhalatorüberwachungsvorrichtung gekoppelt ist, wobei der zentrale Server vorzugsweise so konfiguriert ist, dass er die von der Inhalatorüberwachungsvorrichtung bereitgestellten Feedback-Informationen und/oder die Inhalationseigenschaften des Benutzers mit ergänzenden Daten zusammenführt, um eine Handlungsempfehlung basierend auf den zusammengeführten Inhalationseigenschaften des Benutzers und den ergänzenden Daten zu bestimmen, wobei die ergänzenden Daten vorzugsweise weitere klinische oder physiologische Daten bezüglich des Benutzers, weitere Daten bezüglich des dem Benutzer verabreichten Medikaments und/oder weitere Umgebungsinformationen bezüglich des Ortes, an dem der Benutzer das Inhalatorgerät verwendet hat, und/oder Daten von Drittbenutzern umfassen, und wobei die Handlungsempfehlung vorzugsweise eine Maßnahme bei mangelnder Therapietreue und/oder eine Risikomaßnahme umfasst, wobei eine Maßnahme bei mangelnder Therapietreue vorzugsweise umfasst, wobei der zentrale Server konfiguriert ist, mit dem Computergerät zu kommunizieren, um den Benutzer über eine oder mehrere Maßnahmen zu informieren, die zum Verbessern seiner Inhalationseigenschaften zu ergreifen sind, wobei eine Risikomaßnahme optional umfasst, wobei der zentrale Server konfiguriert ist, mit dem Computergerät zu kommunizieren, um den Benutzer basierend auf seinen Inhalationseigenschaften über sein Risiko einer Verschlechterung oder eine Verbesserung seines medizinischen Zustands zu informieren.

12. Inhalatorüberwachungssystem nach Anspruch 10 oder 11,
wobei der KI-Algorithmus mit den Inhalationseigenschaften des Benutzers über einen bestimmten Zeitraum und/oder mit von dem zentralen Server empfangenen ergänzenden Daten trainiert wird, sodass sich das dem Benutzer bereitgestellte benutzerspezifische Feedback im Laufe der Zeit dynamisch anpasst; und/oder wobei der zentrale Server so konfiguriert ist, dass er einen KI-Algorithmus auf die Inhalationseigenschaften des Benutzers oder auf die Inhalationseigenschaften des Benutzers und die ergänzenden Daten anwendet, wenn der zentrale Server die Handlungsempfehlung bestimmt.

13. Verfahren zum Überwachen einer Inhalatortechnik-Kompetenz und zum Anleiten zu einer optimalen Inhalationstechnik eines Benutzers unter Verwendung eines Inhalatorüberwachungssystems nach einem der vorhergehenden Ansprüche, das Verfahren umfassend:
Empfangen einer oder mehrerer Inhalationseigenschaften eines Benutzers;
Bestimmen von Feedback-Informationen basierend auf der einen oder den mehreren Inhalationseigenschaften des Benutzers; und
Bereitstellen der Feedback-Informationen an den Benutzer;
wobei die Feedback-Informationen eine Benutzerbewertung umfassen, die basierend auf den Inhalationseigenschaften des Benutzers bestimmt werden;
wobei die drahtlosen Übertragungsmittel Daten bezüglich der Inhalationseigenschaften drahtlos an ein externes Computergerät und/oder ein Remote-Computergerät übertragen, und wobei eines oder mehrere der Verarbeitungsmittel, das externe Computergerät und das Remote-Computergerät einen KI-Algorithmus auf die Inhalationseigenschaften anwenden, um das benutzerspezifische Feedback zu bestimmen, wobei der KI-Algorithmus mit den Inhalationseigenschaften des Benutzers trainiert wird, sodass sich das dem Benutzer bereitgestellte benutzerspezifische Feedback im Laufe der Zeit dynamisch anpasst, und
die Feedback-Informationen dem Benutzer bereitstellt, um ihn in Echtzeit zu einer optimalen Inhalationstechnik anzuleiten und seine Inhalationstechnik zu verbessern;
wobei die Inhalationseigenschaften umfassen:
Strömungseigenschaften;
Schwellenwert, Dauer und Intensität des Schüttelns der Inhalatorüberwachungsvorrichtung vor der Abgabe des Medikaments aus dem Inhalator;
Zeit zwischen dem Ende des Schüttelns und der Abgabe des Medikaments;
Zeit zwischen der Abgabe des Medikaments und dem Beginn der Inhalation;
Inhalationsrate;
Volumen des inhalierten Medikaments und
Dosierungsinformationen zu dem Medikament.

14. Verfahren nach Anspruch 13, umfassend das Empfangen einer oder mehrerer Spirometrie-Messungen des Benutzers, wobei die Spirometrie-Messungen den exspiratorischen Spitzenfluss, die forcierte Vitalkapazität und/oder das forcierte exspiratorische Volumen umfassen.

## Revendications

1. Système de surveillance d'inhalateur destiné à surveiller la compétence technique d'inhalateur, à guider vers la technique d'inhalation optimale d'un utilisateur et à améliorer la technique de l'inhalateur comprenant :
un inhalateur (9) conçu pour administrer un médicament ;
une pièce intercalaire (11) ;
un dispositif de surveillance de l'inhalateur (1), un dispositif informatique externe et un dispositif informatique distant ;
dans lequel le dispositif de surveillance de l'inhalateur comprend :
un corps (2) comprenant une entrée (3) et une sortie (5) qui sont en communication fluidique l'une par rapport à l'autre pour définir un canal entre elles ;
dans lequel l'entrée est destinée à être accouplée à l'inhalateur (9) destinée à administrer un médicament et la sortie est destinée à être accouplée à la pièce intercalaire (11) à travers lequel un utilisateur peut inhaler le médicament administré à partir de l'inhalateur ;
un ou plusieurs capteurs (15) qui sont conçus pour détecter et mesurer les caractéristiques d'inhalation lorsque l'utilisateur inhale le médicament administré à partir de l'inhalateur au moyen du dispositif de surveillance de l'inhalateur par l'intermédiaire de la pièce intercalaire en lors de son utilisation ; et
un moyen de traitement qui est conçu pour déterminer des informations de rétroaction en fonction des caractéristiques d'inhalation de l'utilisateur ;
**caractérisé en ce que** le dispositif de surveillance de l'inhalateur comprend des moyens de transmission sans fil configurés pour transmettre des données concernant les caractéristiques d'inhalation au dispositif informatique externe et/ou au dispositif informatique distant, dans lequel un ou plusieurs des moyens de traitement, dispositif informatique externe et dispositif informatique distant est conçu pour appliquer un algorithme d'IA aux données de caractéristiques d'inhalation pour déterminer la rétroaction spécifique de l'utilisateur, dans lequel l'algorithme d'IA est entraîné avec les caractéristiques d'inhalation de l'utilisateur de sorte que la rétroaction spécifique de l'utilisateur fourni à l'utilisateur s'adapte de manière dynamique au fil du temps, et pour fournir les informations de rétroaction à l'utilisateur afin de guider la technique d'inhalation optimale de l'utilisateur et d'améliorer la technique d'inhalateur de l'utilisateur en temps réel ; et
dans lequel les caractéristiques d'inhalation comprennent :
les caractéristiques du débit d'air ;
le seuil, la durée et l'intensité de l'agitation du dispositif de surveillance de l'inhalation, avant l'administration du médicament à partir de l'inhalateur ;
le temps écoulé entre la fin de l'agitation et l'administration du médicament ;
le temps écoulé entre l'administration du médicament et le début de l'inhalation ;
le taux d'inhalation ;
le volume de médicament inhalé ; et
les informations sur la posologie du médicament.

2. Système de surveillance d'inhalateur selon la revendication 1, comprenant en outre un moyen de rétroaction qui est conçu pour fournir une rétroaction visuelle, audible et/ou haptique à l'utilisateur en fonction des informations de rétroaction et/ou d'une ou plusieurs des caractéristiques d'inhalation, de préférence dans lequel le moyen de rétroaction est conçu pour fournir la rétroaction à l'utilisateur en temps réel, et de préférence dans lequel le moyen de rétroaction comprend une pluralité de voyants à DEL qui sont situées sur le corps du dispositif, qui sont conçus pour éclairer selon une séquence prédéfinie en fonction de la ou des caractéristiques d'inhalation de l'utilisateur.

3. Système de surveillance d'inhalateur selon une quelconque revendication précédente, dans lequel le ou les capteurs comprennent : au moins un capteur de pression d'air ; au moins un capteur de mouvement et/ou au moins un capteur environnemental.

4. Système de surveillance d'inhalateur selon une quelconque revendication précédente, dans lequel les informations de rétroaction comprennent au moins les caractéristiques d'inhalation de l'utilisateur.

5. Système de surveillance d'inhalateur selon une quelconque revendication précédente, dans lequel les informations de rétroaction comprennent un score utilisateur qui est déterminé en fonction des caractéristiques d'inhalation de l'utilisateur, de préférence un score utilisateur séparé est déterminé par rapport à chacune des caractéristiques d'inhalation différentes de l'utilisateur, dans lequel le score utilisateur est déterminé en fonction des caractéristiques d'inhalation de l'utilisateur par rapport à un ou plusieurs seuils prédéfinis pour la ou les caractéristiques d'inhalation.

6. Système de surveillance d'inhalateur selon la revendication 5, dans lequel le moyen de traitement est conçu pour surveiller en continu les caractéristiques d'inhalation de l'utilisateur sur une période temporelle et modifier le ou les scores utilisateurs en fonction d'un ou plusieurs changements dans les caractéristiques d'inhalation de l'utilisateur sur la période temporelle et/ou modifier un ou plusieurs seuils prédéfinis pour les caractéristiques d'inhalation en fonction d'un ou plusieurs changements dans les caractéristiques d'inhalation de l'utilisateur sur la période temporelle.

7. Système de surveillance d'inhalateur selon la revendication 5 ou 6, dans lequel les seuils prédéfinis pour la ou les caractéristiques d'inhalation varient en fonction d'un ou plusieurs attributs de l'utilisateur tels que l'âge, le ou les états de santé, le sexe ou tout autre attribut de l'utilisateur approprié.

8. Système de surveillance d'inhalateur selon une quelconque revendication précédente, dans lequel le dispositif de surveillance de l'inhalateur comprend une fonctionnalité de spirométrie permettant d'acquérir et/ou de déterminer les mesures de spirométrie d'un utilisateur ;
dans lequel l'entrée sert à être accouplée à un embout buccal (8) dans lequel l'utilisateur peut expirer ;
et
dans lequel les capteurs sont conçus pour acquérir des données de spirométrie.

9. Système de surveillance d'inhalateur selon la revendication 8, dans lequel les mesures de spirométrie comprennent le débit expiratoire maximal, la capacité vitale forcée et/ou le volume expiratoire.

10. Système de surveillance d'inhalateur selon une quelconque revendication précédente,
dans lequel les informations de rétroaction comprennent des données de milieux fournies à l'utilisateur par le dispositif informatique externe, et/ou dans lequel les informations de rétroaction comprennent un score utilisateur, dans lequel de manière idéale un score utilisateur distinct est déterminé pour chacune des caractéristiques d'inhalation de l'utilisateur, de préférence dans lequel le score utilisateur pour chacune des caractéristiques d'inhalation est pondéré de manière dynamique en fonction d'une ou plusieurs caractéristiques d'inhalation de l'utilisateur.

11. Système de surveillance d'inhalateur selon la revendication 10,
comprenant en outre un serveur central connecté de manière à communiquer avec le dispositif informatique et/ou avec le dispositif de surveillance de l'inhalateur, dans lequel le serveur central est conçu de préférence pour consolider les informations de rétroaction fournies par le dispositif de surveillance de l'inhalateur et/ou les caractéristiques d'inhalation de l'utilisateur avec des données supplémentaires afin de déterminer une action conseillée en fonction des caractéristiques d'inhalation consolidées de l'utilisateur et des données supplémentaires, dans lequel les données supplémentaires comprennent de préférence l'utilisateur concernant le médicament reçu par l'utilisateur, et/ou des informations environnementales supplémentaires concernant l'endroit où l'utilisateur a fait usage de l'appareil inhalateur et/ou des données utilisateur tierces et dans lequel l'action conseillée comprend de préférence une action de non-observance et/ou une action de risque, dans lequel de préférence une action de non-observance consiste en ce que le serveur central est conçu pour communiquer avec le dispositif informatique afin d'informer l'utilisateur d'une ou plusieurs actions à entreprendre pour améliorer ses caractéristiques d'inhalation, dans lequel éventuellement une action de risque consiste en ce que le serveur central est conçu pour communiquer avec le dispositif informatique afin d'informer l'utilisateur de son risque de détérioration ou d'amélioration de son état de santé en fonction de ses caractéristiques d'inhalation.

12. Système de surveillance d'inhalateur selon la revendication 10 ou 11,
dans lequel l'algorithme d'IA est entraîné avec les caractéristiques d'inhalation de l'utilisateur sur une période temporelle et/ou les données supplémentaires reçues du serveur central de sorte que la rétroaction spécifique fournie par l'utilisateur s'adapte de manière dynamique sur la période temporelle ; et/ou dans lequel le serveur central est conçu pour appliquer un algorithme d'IA aux caractéristiques d'inhalation de l'utilisateur ou aux caractéristiques d'inhalation de l'utilisateur et aux données supplémentaires, lorsque le serveur central détermine l'action conseillée.

13. Procédé destiné à surveiller la compétence de technique de l'inhalateur et guider vers la technique d'inhalation optimale d'un utilisateur à l'aide d'un système de surveillance d'inhalateur selon une quelconque revendication précédente, le procédé comprenant :
la réception d'une ou plusieurs caractéristiques d'inhalation d'un utilisateur ;
la détermination des informations de rétroaction en fonction d'une ou plusieurs caractéristiques d'inhalation de l'utilisateur ; et
la fourniture des informations de rétroaction à l'utilisateur ;
dans lequel les informations de rétroaction comprennent un score utilisateur déterminé en fonction des caractéristiques d'inhalation de l'utilisateur ;
dans lequel la transmission sans fil signifie transmettre sans fil des données concernant les caractéristiques d'inhalation à un dispositif informatique externe et/ou à un dispositif informatique distant, et dans lequel un ou plusieurs moyens de traitement, le dispositif informatique externe et le dispositif informatique distant, appliquent un algorithme d'IA aux caractéristiques d'inhalation pour déterminer la rétroaction spécifique à l'utilisateur, où l'algorithme d'IA est entraîné avec les caractéristiques d'inhalation de l'utilisateur de sorte que la rétroaction spécifique fournie à l'utilisateur s'adapte de manière dynamique au fil du temps, et
fournit les informations de rétroaction à l'utilisateur pour guider vers la technique d'inhalation optimale de l'utilisateur et améliorer la technique de l'inhalateur en temps réel ;
dans lequel les caractéristiques d'inhalation comprennent :
les caractéristiques du débit d'air ;
le seuil, la durée et l'intensité de l'agitation du dispositif de surveillance de l'inhalation, avant l'administration du médicament à partir de l'inhalateur ;
le temps écoulé entre la fin de l'agitation et l'administration du médicament ;
le temps écoulé entre l'administration du médicament et le début de l'inhalation ;
le taux d'inhalation ;
le volume de médicament inhalé ; et
les informations sur la posologie du médicament.

14. Procédé selon la revendication 13, qui comprend la réception d'une ou plusieurs mesures spirométriques de l'utilisateur, dans lequel les mesures de spirométrie comprennent le débit expiratoire maximal, la capacité vitale forcée et/ou le volume expiratoire forcé.
